# EUROPÄISCHE PATENTSCHRIFT

(11) **EP 2 844 936 B1**
(45) Veröffentlichungstag und Bekanntmachung des Hinweises auf die Patenterteilung: **12.10.2016**
(21) Anmeldenummer: 13720925.0
(22) Anmeldetag: 02.05.2013
(51) Int. Cl.: F26B 5/06, F26B 25/00, B67B 3/10, B67B 3/14

(54) **VERFAHREN UND VORRICHTUNG ZUR BEHANDLUNG VON BEHÄLTERN UND IN DIESEN AUFBEWAHRTEN SUBSTANZEN FÜR MEDIZINISCHE, PHARMAZEUTISCHE ODER KOSMETISCHE ANWENDUNGEN**
METHOD AND DEVICE FOR TREATING CONTAINERS AND SUBSTANCES STORED THEREIN FOR MEDICAL, PHARMACEUTICAL OR COSMETIC APPLICATIONS
PROCÉDÉ ET DISPOSITIF POUR MANIPULER DES RÉCIPIENTS ET DES SUBSTANCES CONTENUES DANS CES RÉCIPIENTS POUR DES APPLICATIONS MÉDICALES, PHARMACEUTIQUES OU COSMÉTIQUES

(30) Priorität: 03.05.2012 DE 102012103899; 03.05.2012 US 201261642125 P; 13.07.2012 DE 102012106341; 04.09.2012 DE 102012108215; 04.09.2012 US 201261696457 P; 05.11.2012 DE 102012110547
(43) Veröffentlichungstag der Anmeldung: 11.03.2015
(60) Teilanmeldung: 14189484.0
(73) Patentinhaber: Schott AG, 55122 Mainz (DE)
(72) Erfinder: WISSNER, Kai, 69495 Hirschberg (DE); PAWLOWSKI, Edgar, 55271 Stadecken-Elsheim (DE); DEUTSCHLE, Gregor, Fritz, 65185 Wiesbaden (DE); KOCH, Kristopher, Lebanon, Pennsylvania 17042 (US)
(74) Vertreter: 2K Patentanwälte Blasberg Kewitz & Reichel
(86) Internationale Anmeldenummer: PCT/EP2013/059183
(87) Internationale Veröffentlichungsnummer: WO 2013/164422

(56) Entgegenhaltungen:
- WO-A1-2009/015862
- DE-A1- 3 908 582
- DE-A1-102009 027 452
- GB-A- 295 672
- GB-A- 590 421
- US-A- 3 537 189
- US-A- 5 964 043

## Beschreibung

### Gebiet der Erfindung

Die vorliegende Erfindung betrifft die gleichzeitige Behandlung oder Weiterverarbeitung von Behältern, die zur Aufbewahrung von Substanzen für kosmetische, medizinische oder pharmazeutische Anwendungen dienen oder diese enthalten sollen, durch Gefriertrocknung, insbesondere von Fläschchen mit Wirkstoffen oder Wirkstofflösungen, und betrifft ferner die gleichzeitige automatisierte Förderung und Übergabe einer Mehrzahl von Behältern an Prozessstationen, beispielsweise einer Abfüll- oder Bearbeitungsanlage, eines Steriltunnels, eines Gefriertrockenschranks zum Lyophilisieren einer wirkstoffhaltigen Flüssigkeit oder dergleichen.

### Hintergrund der Erfindung

Als Behälter zur Aufbewahrung und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die Haltestruktur aus dem Transport- und Verpackungsbehälter entnommen und die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten wird. Die einzelnen Medikamentenbehälter werden zunächst lose in Aufnahmen, die in der Haltestruktur ausgebildet sind, angeordnet. Anschließend wird die Haltestruktur in den Transport- und Verpackungsbehälter eingesetzt und dieser mit einem gasundurchlässigen Kunststoffschlauch umgeben. Beim anschließenden Evakuieren der so ausgebildeten Verpackungseinheit wird der Kunststoffschlauch aufgrund des in dem Schlauch vorherrschenden Unterdrucks in die Zwischenräume zwischen den Medikamentenbehältern hineingedrückt, was so einerseits zu einer Stabilisierung der Position der Medikamentenbehälter in der Haltestruktur führt und andererseits eine weitere unkontrollierte Kollision von benachbarten Medikamentenbehältern verhindert. Beim Evakuieren und beim anschließenden Öffnen des Kunststoffschlauchs können jedoch die Medikamentenbehälter seitlich verrutschen, was den Automatisierungsaufwand zur Weiterverarbeitung der Medikamentenbehälter erhöht. Ferner können die Medikamentenbehälter nach dem Öffnen des Kunststoffschlauchs dennoch unkontrolliert kollidieren, was die vorgenannten Nachteile mit sich bringt. Die Medikamentenbehälter können nicht in dem Transport- oder Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1, US 2011/0277419 A1, WO 2012/025549 A1, WO 2011/015896 A1, WO 2012/007056 A1 und WO 2009/015862 A1 offenbart.

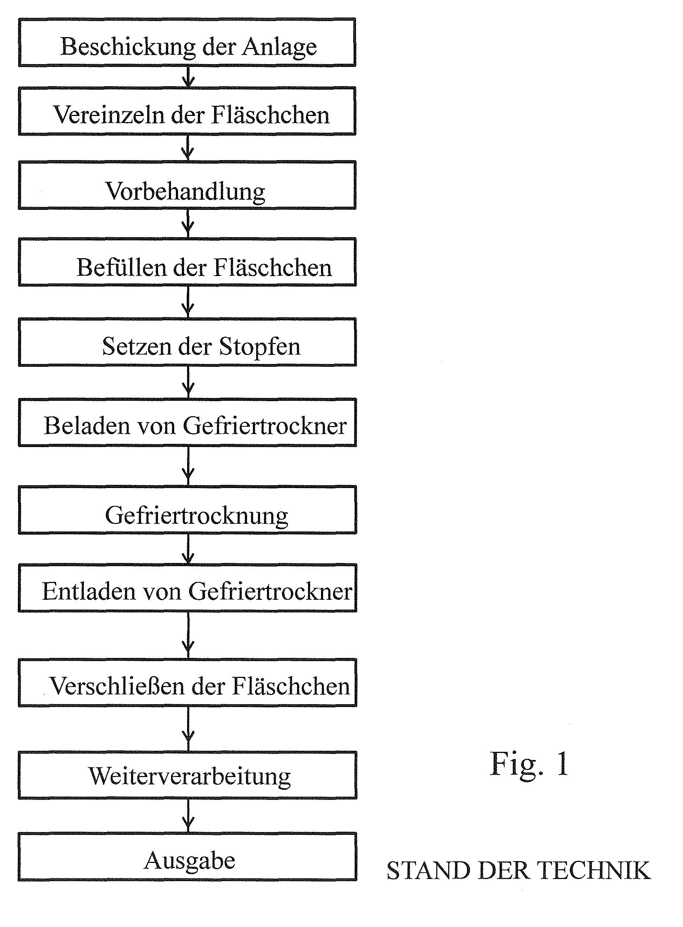

US 5 964 043 A offenbart ein Verfahren bzw. eine Vorrichtung zur Behandlung oder Verarbeitung von Behältern gemäß dem Oberbegriffs von Anspruch 1 bzw. 16, bei dem die Medikamentenbehälter zur Weiterverarbeitung vereinzelt werden müssen. Diese Vorgehensweise sei beispielhaft anhand der Fig. 1 erläutert, die ein schematisches Flussdiagram eines herkömmlichen Verfahrens zur Gefriertrocknung von pharmazeutischen Präparaten in Medikamentenbehältern darstellt, wie in der US 5 964 043 A offenbart.

Zunächst wird die Prozessanlage, ein Steriltunnel, mit den Fläschchen beschickt. Hierzu werden die Fläschchen kopfüber in Transportrahmen eingehängt, die dann durch die Prozessanlage gefördert werden. Zur Vorbehandlung werden die in dem Transportrahmen gehaltenen Fläschchen zunächst sterilisiert. Anschließend werden die Transportrahmen mit den darin aufbewahrten Fläschchen gewendet und mit einer Wirkstofflösung befüllt. Anschließend wird auf den oberen Rand der Fläschchen ein Stopfen aufgesetzt, in welchem ein Kanal ausgebildet ist, über den der Flascheninnenraum während der Gefriertrocknung jeweils mit der Kammer des Gefriertrockenschranks kommuniziert.

Zur Gefriertrocknung (auch als Lyophilisation oder Sublimationstrocknung bezeichnet) werden die Fläschchen dann aus dem Transportrahmen entnommen und einzeln in den Gefriertrockenschrank eingebracht. Dabei müssen die Böden der Fläschchen zur Erzielung eines guten Kühleffekts unmittelbar auf einem planar ausgebildeten Kühlboden abgesetzt werden. Wird an dieser Stelle kein vollflächiger unmittelbarer Kontakt gewährleistet, so führt dies zu einer erheblichen Verlängerung des Gefriertrocknungsprozesses, was zu höheren Kosten führt.

Nach der Gefriertrocknung werden die Fläschchen aus dem Gefriertrockenschrank entnommen, die Stopfen heruntergedrückt und ein Metalldeckel auf den Stopfen aufgesetzt und gebördelt. Derart verarbeitete Fläschchen werden dann ausgeliefert, beispielsweise indem eine Mehrzahl von Fläschchen gemeinsam von einem Träger aufgenommen und der Träger in einen Transport- und Verpackungsbehälter eingesetzt wird, der zur Auslieferung dann steril verpackt wird.

Der für die Gefriertrocknung notwendige unmittelbare Kontakt zwischen dem Boden der Medikamentenbehälter und dem Kühlboden erfordert herkömmlich eine Behandlung oder Verarbeitung einzelner Behälter, was die Verarbeitungs- und Verpackungskosten erhöht. Eine chargenweise Weiterverarbeitung von Medikamentenbehälter ist herkömmlich nicht möglich. Jedenfalls ist ein unmittelbarer Kontakt der Böden der Medikamentenbehälter, insbesondere der Böden von Fläschchen, bei den herkömmlichen Haltestrukturen nicht möglich.

### Zusammenfassung der Erfindung

Aufgabe der vorliegenden Erfindung ist es, ein Verfahren zur Behandlung oder Verarbeitung von Behältern, die zur Aufbewahrung von Substanzen für kosmetische, medizinische oder pharmazeutische Anwendungen dienen, einschließlich einer Gefriertrocknung dahingehend weiterzubilden, dass dieses noch rascher und wirtschaftlicher, besser automatisierbar und zuverlässiger ausgeführt werden kann. Gemäß einem weiteren Gesichtspunkt der vorliegenden Erfindung soll eine entsprechend ausgelegte Vorrichtung zur Behandlung oder Verarbeitung solcher Behälter einschließlich einer Gefriertrocknung bereitgestellt werden.

Diese Aufgabe wird gemäß der vorliegenden Erfindung durch ein Verfahren mit den Merkmalen nach Anspruch 1 sowie durch eine Vorrichtung nach Anspruch 16 gelöst. Weitere vorteilhafte Ausführungsformen sind Gegenstand der rückbezogenen Unteransprüche.

Bei einem Verfahren zur Behandlung oder Verarbeitung von Behältern, die zur Aufbewahrung von Substanzen für kosmetische, medizinische oder pharmazeutische Anwendungen dienen oder diese enthalten und die an einem Ende offen ausgebildet sind, insbesondere von Fläschchen, werden die Behälter mittels einer Fördereinrichtung zur Behandlung oder Verarbeitung automatisiert an Prozessstationen vorbeigeführt oder durchlaufen diese, wobei eine Mehrzahl von Behältern von der Fördereinrichtung gefördert wird, während diese von einem Träger gemeinsam in einer regelmäßigen zweidimensionalen Anordnung gehalten werden, und wobei der Träger eine Mehrzahl von Öffnungen oder Aufnahmen aufweist, welche die regelmäßige Anordnung vorgeben. Dabei sind die Böden der Behälter an deren geschlossenen Enden von einer ersten Seite des Trägers her frei zugänglich. Erfindungsgemäß ist eine Prozessstation ein Gefriertrockenschrank, mit zumindest einer Kühlfläche, in welchem ein Gefriertrocknungsprozess ausgeführt wird, wobei die Böden der Behälter während des rocknungsprozesses in dem Gefriertrockenschrank in einem unmittelbaren Kontakt zu der jeweiligen Kühlfläche stehen, während die Behälter von dem Träger gehalten werden oder in den Öffnungen oder Aufnahmen des Trägers aufgenommen sind.

Somit können die Behälter erfindungsgemäß chargenweise behandelt oder verarbeitet werden. Einer die Prozesse aufwändig machenden Entnahme aus dem Träger und einer Vereinzelung bedarf es erfindungsgemäß nicht. Hierzu ist der Träger so ausgelegt, dass die Behälter an diesem form- oder reibschlüssig gehalten werden können. Insbesondere werden die Behälter in hierfür geeignet ausgebildeten Öffnungen oder Aufnahmen des Trägers gehalten.

Bei dem Verfahren sind die Böden der Behälter, während diese an dem Träger gehalten werden, jedenfalls größtenteils zugänglich, sodass die Böden der Behälter beispielsweise auf einem Kühlboden eines Gefriertrockenschranks abgesetzt werden können und ein vollflächiger Kontakt für eine effiziente Kühlung gewährleistet ist. Zweckmäßig sind hierzu sämtliche hälter an dem Träger auf "gleicher Höhe" gehalten.
Damit die der Behälter vollständig oder zum überwiegenden Teil zugänglich sind, sind diese nicht von einer Haltestruktur oder dergleichen abgedeckt, während diese an dem Träger gehalten werden.

Weiterhin können auch die Seitenwandabschnitte der Behälter, während diese an dem Träger gehalten werden, jedenfalls größtenteils zugänglich sein, sodass die Behälter an dem Träger einfach gehandhabt werden können.

Die Träger können gemäß einer weiteren Ausführungsform so ausgelegt sein, dass die Behälter, während diese an diesen gehalten werden, verschoben oder gedreht oder in vergleichbarer Weise verstellt oder bewegt werden können. Dies lässt sich durch entsprechende Auslegung des Form- oder Reibschlusses in einfacher Weise gewährleisten. Somit können die Behälter beispielsweise zum Bördeln eines Metalldeckels, der auf deren oberen Rand aufgesetzt ist, gedreht werden, während diese an dem Träger gehalten sind.

Gemäß einer weiteren Ausführungsform können die Behälter zur Behandlung oder Verarbeitung an oder in der Prozessstation in der jeweiligen Öffnung oder Aufnahme des Trägers in Längsrichtung in eine angehobene Position verschoben werden, in welcher die weitere Behandlung oder Verarbeitung dann erleichtert ist. Beispielsweise kann in dieser angehobenen Position der Boden der Behälter vollständig zugänglich sein oder der obere Rand der Behälter über den oberen Rand des Trägers oder eines Transport- und Verpackungsbehälters geeignet weit vorstehen, sodass erst in der angehobenen Position eine Behandlung oder Verarbeitung möglich ist.

Zweckmäßig werden die Behälter in dieser angehobenen Position im Bereich Ihrer zylindrischen Seitenwand oder aber eines verengten Halsabschnitts unterhalb des oberen Rands oder an ihrem oberen Rand gehalten, was von der jeweiligen Prozessstation abhängen kann.

Gemäß einer weiteren Ausführungsform sind die Behälter in der angehobenen Position weiterhin in den Öffnungen oder Aufnahmen des Trägers aufgenommen, werden jedoch auf einer zusätzlichen Abstützfläche abgestützt oder mittels einer zusätzlichen Halte- oder Greifeinrichtung gehalten, während diese an oder in der Prozessstation behandelt oder verarbeitet werden. Die Haltemittel an dem Träger sind also so ausgelegt, dass diese in der angehobenen Position die Behälter nicht halten, jedenfalls nicht mit einer dem Gewicht der Behälter entsprechenden ausreichenden Haltekraft. Jedoch brauchen auch bei dieser Ausführungsform die Behälter nicht vollständig aus dem Träger entnommen zu werden, sodass diese weiterhin chargenweise behandelt oder verarbeitet werden können, jedoch dennoch rascher an einen nachfolgenden Prozessschritt übergeben werden können. Bei der vorgenannten Halte- oder Greifeinrichtung kann es sich beispielsweise um einen Roboterarm einer volllautomatisch gesteuerten Prozessanlage handeln.

Bei der vorgenannten Abstützfläche kann es sich insbesondere auch um Führungsflächen handeln, die die weitere Förderung der Behälter durch die Prozessanlage geeignet führen. Diese Führungsflächen können beispielsweise auch kurven- oder rampenförmig ausgebildet sein, um so Höhenlagen der Behälter während ihrer Förderung durch die Prozessanlage geeignet vorzugeben. Insbesondere kann eine solche Abstützfläche auch mit einem Drehteller versehen oder als solcher ausgebildet sein, um einzelne Behälter zu drehen, während diese auch weiterhin in den Öffnungen oder Aufnahmen des Trägers aufgenommen sind. Zweckmäßig ist hierzu der Träger so ausgelegt, dass die von den Haltemitteln ausgeübte Haltekraft in einfacher Weise verstellt werden kann, nämlich zwischen einer ersten Haltestellung, in der die Behälter mit ausreichender Kraft form- oder reibschlüssig an dem Träger gehalten sind, und einer zweiten Haltestellung, in der die Haltekraft vollständig oder jedenfalls in ausreichendem Maße reduziert ist. Dies kann beispielsweise durch Verstellung der Öffnungsweite der Öffnungen oder Aufnahmen des Trägers in einfacher Weise bewerkstelligt werden.

Gemäß einer weiteren Ausführungsform können Randabschnitte des Trägers, insbesondere einer Grundplatte desselben, abgenommen oder weggeschwenkt werden, um die Grundfläche des Trägers insgesamt zu verringern, wenn die Behälter in oder an der Prozessstation behandelt oder verarbeitet werden. Dies führt insbesondere bei der Gefriertrocknung einer Mehrzahl von in einem Träger gehaltenen Behältern zu bedeutenden Kosteneinsparungen.

Gemäß einem ersten Gesichtspunkt der vorliegenden Erfindung sind die Behälter (container) in dem Träger (Haltestruktur) reibschlüssig gehalten. Zur kraft- bzw. reibschlüssigen Halterung von zylindrischen Behältern steht eine Vielzahl von Haltemitteln zur Verfügung. Kraft- bzw. reibschlüssige Verbindungen setzen bekanntermaßen nur eine ausreichende NormalKraft auf die miteinander zu verbindenden Flächen voraus. Die gegenseitige Verschiebung zwischen Behälter und Träger ist also verhindert solange die durch die Haftreibung zwischen Träger und Behälter bewirkte Gegenkraft nicht überschritten wird. Der Kraft- bzw. Reibschluss ist verloren und die Flächen rutschen aufeinander, wenn die tangential wirkende Last-Kraft größer als die Haftreibungs-Kraft ist. Letzteres ist jedoch bei den vergleichsweise geringen Gewichten der in dem Träger aufzunehmenden Behälter unwahrscheinlich, kann jedoch ausgenutzt werden, um die Behälter, während diese in dem Träger aufbewahrt sind, von einer ersten Stellung axial in eine zweite Stellung zu verschieben, in welcher diese weiterverarbeitet werden können, beispielsweise deren Öffnungen mit einem Stopfen verschlossen werden oder auf den Stopfen ein äußerer Verschluss (beispielsweise Bördelkappe oder Krampe), häufig aus Aluminiumblech, aufgebracht wird.

Der Kraft- bzw. Reibschluss erfolgt zweckmäßig entweder unterhalb des aufgeweiteten oberen Randes der Behälter, d.h. an ihrem verengten Hals- bzw. Nackenbereich unterhalb des oberen Rands, oder im Bereich der zylindrischen Seitenwand. Einer bodenseitigen Abstützung der Behälter bedarf es erfindungsgemäß grundsätzlich nicht, sodass ein Zugriff auf die Unterseiten (Böden) der in dem Träger gehaltenen Behälter grundsätzlich möglich ist. Dies ermöglicht erfindungsgemäß, dass die Behälter, während diese in dem Träger aufgenommen sind, weiterverarbeitet werden können, wie nachfolgend ausgeführt. Mit anderen Worten, die Behälter können in Trägern chargenweise weiter verarbeitet werden, bleiben jedoch während der Weiterverarbeitung weiterhin zuverlässig und kollisionsfrei in oder an den Trägern gehalten, was erhebliche Geschwindigkeitsvorteile und Vorteile bei der Automatisierung von Weiterverarbeitungsanlagen ermöglicht und so insgesamt zu noch wirtschaftlicheren und kostengünstigeren Prozessen führt. Ferner ist ein unmittelbarer Glas-Glas-Kontakt von benachbarten Behältern zuverlässig verhindertt, was Abrieb und Verunreinigungen innerhalb der Weiterverarbeitungsanlage wirkungsvoll verhinder und so erheblich längeren Laufzeiten und Wartungsintervalle der Anlagen ermöglicht. Ferner können Kratzer oder die Erzeugung von Partikeln auf oder in den Behältern wirkungsvoll verhindert werden.

Der bei dem erfindungsgemäßen Verfahren verwendete Träger ermöglicht dabei zweckmäßig die Entnahme der Behälter nach oben oder unten. Da die Position des Kraft- bzw. Reibschlusses zwischen Behälter und Haltestruktur einfach variiert werden kann, ist die Haltestruktur sehr flexibel auch für Behälter mit unterschiedlichen Außenabmessungen einsetzbar, solange eine ausreichende Normalkraft für den Reibschluss gewährleistet werden kann. Insbesondere können die Behälter in dem Träger in axialer Richtung einfach verschoben werden, sodass Behälter mit unterschiedlichen Höhen in oder an demselben Träger gehalten werden können. Die axiale Verschieblichkeit der Behälter ermöglicht auch einen einfachen Toleranzausgleich.

Gemäß einem zweiten Gesichtspunkt der vorliegenden Erfindung sind die Behälter (container) in der Haltestruktur formschlüssig gehalten. Zur formschlüssigen Halterung von zylindrischen Behältern steht eine Vielzahl von Haltemitteln zur Verfügung. Die gegenseitige Verschiebung zwischen Behälter und Haltestruktur ist verhindert, solange ein Verbindungspartner dem anderen Verbindungspartner im Wege steht, diesen also sperrt.

Der Formschluss erfolgt zweckmäßig entweder unterhalb des aufgeweiteten oberen Randes der Behälter, d.h. im Bereich des verengten Hals- bzw. Nackenbereichs und unmittelbar unterhalb des oberen Rands, oder am unteren Ende der Behälter, beispielsweise an einem Boden der Behälter. Zweckmäßig ist der aufgeweitete obere Rand oder das untere Ende bzw. der Boden der Behälter auf den Formschtusselementen der Haltestruktur unmittelbar abgestützt. Alternativ kann der obere Rand oder das untere Ende bzw. der Boden der Behälter auch formschlüssig umgriffen oder hintergriffen werden.

Gemäß einer weiteren Ausführungsform wird der vorgenannte Formschluss insbesondere mittels Haltemitteln ausgebildet, wobei als Haltemittel zumindest zwei Haltezungen an dem Träger bzw. der Haltestruktur vorgesehen sind, die am Rand einer jeweiligen Öffnung oder Aufnahme vorgesehen sind und von einer Oberseite des Trägers abragen, um den jeweiligen Behälter in der Öffnung oder Aufnahme zu halten. Dabei sind die Haltezungen so ausgelegt, dass diese beim Einführen der Behälter in die Öffnungen oder Aufnahmen elastisch weggeschwenkt oder weggeklappt werden, und ferner so auf die Behälter abgestimmt, dass diese mit radialem Spiel von den Haltezungen gehalten sind. Das radiale Spiel ermöglicht, dass Behälter mit unterschiedlichen radialen Toleranzen und/oder Außenabmessungen von derselben Haltestruktur zuverlässig gehalten werden können. Das radiale Spiel ist zweckmäßig so ausgelegt und auf die Außenkontur und -abmessung der Behälter abgestimmt, dass niemals gleichzeitig sämtliche Haltezungen den verengten Halsabschnitt am oberen Ende der Behälter, insbesondere Fläschchen, berühren. Gleichzeitig verhindert das radiale Spiel auch ein unerwünschtes Verspannen oder gar Aufwölben des Trägers beim Halten von Behältern mit unterschiedlichen radialen Toleranzen und/oder Außenabmessungen, was erhebliche Vorteile insbesondere bei der gleichzeitigen Prozessierung einer Mehrzahl von Behältern, während diese von der Haltestruktur gehalten sind, bietet, beispielsweise bei der Gefriertrocknung bei Prozessierung bei sehr niedrigen Temperaturen.

Selbst wenn sich der Träger dennoch bei der Prozessierung verziehen oder aufwölben sollte, kann dennoch ein gleichmäßiger Bodenkontakt zu sämtlichen von der Haltestruktur gehaltenen Behältern realisiert werden, insbesondere wenn diese ergänzend mit einem ausreichendem axialen Spiel von den Haltezungen an der Haltestruktur gehalten sind, da das axiale Spiel zusätzlich auch einen Längentoleranzausgleich ermöglicht.

Die Haltezungen sind dabei ausreichend elastisch ausgebildet oder gelagert, sodass die Behälter axial, d.h. in Richtung der Längsachse der Behälter und senkrecht zur Ebene des Trägers, von der Ober- oder Unterseite des Trägers her in die Öffnungen oder Aufnahmen eingeschoben werden können, insbesondere unter elastischer Verformung der Haltezungen, beispielsweise unter Wegbiegen derselben. Die Bestückung des Trägers mit Behältern kann somit einfach automatisiert werden, was durch eine regelmäßige Anordnung der Öffnungen oder Aufnahmen, bevorzugt in einer zweidimensionalen Matrix, noch weiter begünstigt wird.

Als bevorzugte Stelle, an der die Behälter an den Haltezungen gehalten oder abgestützt sind, hat sich die Unterseite eines verbreiterten oberen Randabschnittes der Behälter erwiesen, wie diese insbesondere bei Fläschchen typischerweise als sog. Rollrand oder Schulter vorgesehen sind. In diesem Bereich steht eine Abstütz- oder Lagerfläche zum Halten oder Abstützen der Behälter mit einer ausreichenden Erstreckung in Radialrichtung der Öffnungen oder Aufnahmen zur Verfügung, um das vorgenannte radiale Spiel bei der Halterung der Behälter ohne Weiteres zu realisieren.

Weil die Behälter in den Öffnungen oder Aufnahmen mit sehr geringem Kraftaufwand angehoben oder bewegt, beispielsweise gedreht, werden können, können diese, während diese sich in der Haltestruktur befinden und von dieser gehalten oder zumindest geführt werden, ohne weiteres bearbeitet werden. Als besonders vorteilhaft hat sich diese Art der Halterung z.B. beim Verschließen der Behälter durch Bördeln eines Metalldeckels erwiesen. Die hierzu erforderlichen Vorgänge können an dem Metalldeckel ausgeführt werden, während der Behälter in der Öffnung oder Aufnahme der Haltestruktur gehalten oder zumindest geführt ist. Als besonders vorteilhaft hat sich diese Art der Halterung auch bei der Prozessierung von Behältern erwiesen, während diese in der Haltestruktur gehalten bzw. aufgenommen sind. Beispielsweise können die Haltestrukturen mit den darin aufgenommenen bzw. gehaltenen Behältern in einen Gefriertrockenschrank eingebracht werden. Aufgrund der Halterung der Behälter mit einem gewissen Spiel in den Haltestrukturen kann gewährleistet werden, dass die Böden von sämtlichen Behältern auf einer kühlenden Unterlage, beispielsweise einem Kühlfinger des Gefriertrockenschranks, gleichmäßig aufliegen. Oder die Behälter können ohne größeren Kraftaufwand in den Öffnungen oder Aufnahmen der Haltestruktur angehoben und zur Prozessierung gehandhabt werden.

Gemäß einer bevorzugten Ausführungsform sind die Haltezungen als elastische Haltezungen ausgebildet, verfügen jedoch über eine ausreichende Elastizität, um beim Einführen der Behälter in die Öffnungen oder Aufnahmen ausreichend elastisch weggeschwenkt oder weggeklappt zu werden, um den Behältern den Weg in die Öffnungen oder Aufnahmen freizugeben. Dies lässt sich durch geeignete Dimensionierung, Materialwahl und Auslegung der Materialstärke der Haltezungen ohne weiteres erreichen. Bevorzugt sind die Haltezungen somit aus einem Kunststoff ausgebildet.

Gemäß einer Ausführungsform sind die Haltezungen elastisch gegen eine Haltestellung vorgespannt, bevorzugt mittels eines elastischen Rückstellelements, beispielsweise einer Rückstellfeder oder eines Kunststoffblättchens oder elastischen Kunststoffgebildes, das mit der zugeordneten Haltezunge geeignet zusammenwirkt und auf der Oberseite des Trägers vorgesehen oder ausgebildet ist.

Gemäß einer Ausführungsform sind die Haltezungen so auf die Behälter abgestimmt, dass die Behälter mit einem verbreiterten Rand, der an einem oberen Ende der Behälter ausgebildet ist, also insbesondere mit dem vorgenannten Rollrand, lose auf Oberseiten der Haltezungen aufliegen. Die Behälter können somit ohne Widerstand nach oben wieder aus den Öffnungen oder Aufnahmen entnommen werden.

Gemäß einer Ausführungsform umgreifen die Haltezungen den verbreiterten Rand dergestalt, dass die Behälter mit radialem Spiel oder mit radialem und axialem Spiel von den Haltezungen gehalten sind. Auf diese Weise können die Behälter in den Öffnungen oder Aufnahmen axial verliersicher gehalten werden. Zum Entnehmen der Behälter aus den Öffnungen oder Aufnahmen brauchen die Haltezungen nur wiederum in der Art, wie beim Einführen der Behälter, zurückgeschwenkt oder zurück geklappt werden.

Gemäß einer Ausführungsform sind die Haltezungen so verteilt auf der Oberseite des Trägers angeordnet, dass diese beim Wegschwenken oder Wegklappen einander nicht unmittelbar berühren und eine unmittelbar benachbarte Öffnung oder Aufnahme nicht versperren. Somit kann die Packungsdichte der Behälter an dem Träger noch weiter erhöht werden. Insbesondere sind die Haltezungen so ausgelegt, dass unmittelbar benachbarte Haltezungen, wenn diese beim Einführen der Behälter in die zugeordneten Öffnungen oder Aufnahmen zum Träger hin geschwenkt oder geklappt werden, einander nicht berühren.

Gemäß einer Ausführungsform sind am oberen Ende der Haltezungen Einführschrägen ausgebildet, welche jeweils in eine von den Haltezungen radial einwärts vorstehende Haltenase zum Halten der Behälter übergehen. Die Behälter können somit noch einfacher und kraftärmer in die Öffnungen oder Aufnahmen eingeführt werden. Insbesondere geraten beim Einführen der Behälter von oben her in die Öffnungen oder Aufnahmen zunächst die Böden bzw. unteren Enden der Behälter in Anlage zu den Einführschrägen. Beim weiteren Einführen der Behälter gleitet das untere Ende bzw. der Boden der Behälter entlang den Einführschrägen abwärts und spreizt dabei die Haltezungen auseinander oder klappt bzw. schwenkt diese zurück. Beim weiteren Einführen der Behälter gerät schließlich die zylindrische Seitenwand in Anlage zu den Haltenasen und gleitet an diesen entlang, solange bis schließlich die Unterseite des vorgenannten Rollrands lose auf den Haltenasen der Haltezungen aufliegt.

Bei dem vorgenannten Verfahren kann eine Haltestruktur zum gleichzeitigen Halten der Mehrzahl von Behältern verwendet werden, die einen Träger, der eine Mehrzahl von Öffnungen oder Aufnahmen aufweist, in welche die Behälter einführbar sind, sowie Haltemittel zum Halten der Behälter in den Öffnungen oder Aufnahmen aufweist, wobei die Haltestruktur eine Längsrichtung (x) und eine Querrichtung (y) aufweist. Dabei können jeweils unmittelbar benachbarte Haltestrukturen so miteinander unmittelbar verbunden werden, dass diese relativ zueinander in der Längsrichtung und/oder in der Querrichtung unverschieblich sind. Mit anderen Worten: die jeweils unmittelbar benachbarten Haltestrukturen können gemeinsam, quasi als Einheit, bestehend aus mehreren (zumindest zwei) Haltestrukturen gehandhabt werden, ohne dass sich deren Lage relativ zueinander wesentlich verändern würde.

Zu diesem Zweck wird eine lösbare, vorübergehende Verbindung der unmittelbar benachbarten Haltestrukturen gewählt, wobei grundsätzlich beliebige form- oder kraftschlüssige Verbindungstechniken eingesetzt werden können, solange die durch die Verbindung erzielbare Verbindungskraft größer ist als die üblicherweise bei der Handhabung oder Prozessierung der Haltestrukturen auftretenden Kräfte, die danach trachten, die unmittelbar benachbarten Haltestrukturen wieder voneinander zu trennen. Die gewählte Verbindungstechnik kann dabei sehr wohl ein gewisses Spiel zwischen den unmittelbar benachbarten Haltestrukturen zulassen, um eine übermäßige Materialbelastung zu vermeiden. Insbesondere können die zur Verbindung vorgesehenen form- oder kraftschlüssig wirkenden Verbindungsstrukturen eine gewisse Elastizität zwischen den unmittelbar benachbarten Haltestrukturen ermöglichen, was sich durch geeignete Auslegung der Verbindungsstrukturen ohne weiteres erzielen lässt.

Durch die vorgenannte lösbare, vorübergehende Verbindung kann insbesondere eine Mehrzahl von Haltestrukturen in einer Reihe hintereinander oder nebeneinander angeordnet, miteinander verbunden und gemeinsam in eine Bearbeitungs- oder Prozessstation, wie beispielweise einen Gefriertrockner, eingebracht und wieder aus diesem entnommen werden. Die Beschickung von Bearbeitungs- oder Prozessstationen, wie beispielsweise Gefriertrocknern, kann manuell aber auch mittels geeigneter Bewegungseinrichtungen halb-automatisch oder vollautomatisch erfolgen. Die Beschickung eines Gefriertrockners kann erfindungsgemäß insbesondere von außen und innen erfolgen.

Gemäß einer weiteren Ausführungsform erfolgt die lösbare, vorübergehende Verbindung der unmittelbar benachbarten Haltestrukturen mittels eines Formschlusses durch Formschlussgebilde, die entlang den Rändern der Haltestrukturen geeignet angeordnet sind und ausgelegt sind, um geeignet miteinander zusammenwirken zu können, um eine lösbare Verbindung zu bewerkstelligen. Der Formschluss wird dabei bevorzugt unmittelbar zwischen den Formschlussgebilden realisiert, d.h. ohne Vermittlung eines dritten Verbindungselements, wie beispielsweise einer Schraube, sodass die Verbindung zeit- und kostensparend realisiert werden kann. Zu diesem Zweck können an einander gegenüber liegenden Rändern der unmittelbar benachbarten Haltestrukturen zueinander korrespondierend ausgebildete Formschlussgebilde ausgebildet sein, die miteinander in einen formschlüssigen Eingriff überführbar sind.

Die Formschlussgebilde können insbesondere für eine Verbindung nach Art einer Schwalbenschwanzverbindung, einer Nut-Feder-Verbindung oder einer Passfeder ausgelegt sein. Denkbar sind auch Aussparungen, beispielweise mit kreisförmigem Querschnitt, in welche korrespondierend ausgebildete stiftartige Vorsprünge einer benachbarten Haltestruktur formschlüssig eingreifen.

Gemäß einer weiteren Ausführungsform sind die Formschlussgebilde als Vorsprünge und Aussparungen entlang den einander gegenüberliegenden Rändern der beiden unmittelbar benachbarten Haltestrukturen ausgebildet, deren Grundflächen, jeweils in Draufsicht betrachtet, verschieden zu einer Rechteckform sind und die unmittelbar korrespondierend zueinander ausgebildet sind. Die Formschlussgebilde können somit in einfacher Weise unmittelbar ineinander gehakt werden. Bevorzugt stehen dabei diese Vorsprünge und Aussparungen nicht aus der von der flächigen Haltestruktur aufgespannten Ebene wesentlich hervor, sodass die Haltestrukturen auch weiterhin flach und somit raumsparend ausgebildet sind. Die vorgenannte Verhakung erfolgt dabei durch einfaches Anheben einer Haltestruktur und anschließendes Absenken, um den vorgenannten Formschluss zwischen den korrespondierend ausgebildeten Formschlussgebilden zu bewerkstelligen. Beispielsweise können die Vorsprünge und Aussparungen eine im Wesentlichen dreieckförmige Grundfläche aufweisen. Bevorzugt sind diese Vorsprünge und Aussparungen alternierend und in regelmäßigen Abständen zueinander entlang von einander gegenüber liegenden Rändern der Haltestrukturen angeordnet, sodass die Haltestrukturen grundsätzlich auch nicht zueinander fluchtend in einer Reihe nebeneinander miteinander verbunden werden können, was beispielsweise zur effektiveren Nutzung von Bearbeitungs- und Prozessstationen mit nicht-rechteckförmiger Grundfläche vorteilhaft sein kann. Die Beschickung von Bearbeitungs- und Prozessstationen kann somit noch flexibler erfolgen.

Gemäß einer weiteren Ausführungsform sind entlang Rändern der zueinander korrespondierend ausgebildeten Vorsprünge und Aussparungen zumindest abschnittsweise Seitenwände ausgebildet, die rechtwinklig von einer Oberfläche der Haltestrukturen abragen. Vorteilhaft ist, dass durch diese vorstehenden Ränder die Kontaktfläche beim Schieben und Ziehen vergrößert ist. Die Ränder wirken dabei quasi als Anschlag- und Führungsflächen und ermöglichen einen noch präziseren Formschluss zwischen den unmittelbar benachbarten Haltestrukturen. Insbesondere kann die Gefahr eines "übereinander Schichtens" der flächigen Haltestrukturen wirkungsvoll reduziert werden.

Gemäß einer weiteren Ausführungsform weisen die Formschlussgebilde an einer ersten der beiden unmittelbar benachbarten Haltestrukturen eine elastische Zunge mit einem darauf ausgebildeten Rastvorsprung oder einer darauf ausgebildeten Rastaussparung sowie an der zweiten der beiden unmittelbar benachbarten Haltestrukturen eine korrespondierend zu dem Rastvorsprung ausgebildete Aufnahme oder einen korrespondierend zu der Rastaussparung ausgebildeten Vorsprung auf. Zum Verbinden werden die Haltestrukturen aufeinander zu bewegt, bis schließlich das vordere Ende der elastischen Zunge in Anlage mit dem Rand der benachbarten Haltestruktur gelangt. Bei der weiteren Annäherung gleitet schließlich die Unterseite der elastischen Zunge auf der Oberfläche der benachbarten Haltestruktur entlang, wobei in diesem Zustand die elastische Zunge geringfügig nach oben gebogen ist. Schließlich greifen der Rastvorsprung und die korrespondierend ausgebildete Aufnahme formschlüssig ineinander ein und kehrt die elastische Zunge zurück in ihren entspannten Ruhezustand, wobei aufgrund des Formschlusses zwischen dem Rastvorsprung und der korrespondierend ausgebildeten Aufnahme eine zuverlässige Verbindung zwischen den benachbarten Haltestrukturen realisiert ist. Die Verbindung und Lösung der Verbindung ist vorteilhaft einfach.

Ein weiterer Gesichtspunkt der vorliegenden Erfindung betrifft ferner einen Transport- und Verpackungsbehälter mit zumindest einer Haltestruktur, wie vorstehend ausgeführt und nachfolgend weiter im Detail offenbart.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1: ein Flussdiagramm eines herkömmlichen Verfahrens zur Behandlung oder Verarbeitung von Behältern;
- Fig. 2a - 2c: einen Transport- und Verpackungsbehälter gemäß einer ersten Ausführungsform zur Verwendung bei einem Verfahren gemäß der vorliegenden Erfindung;
- Fig. 3a - 9e: Transport- und Verpackungsbehälter gemäß einer weiteren Ausführungsformen zur Verwendung bei einem Verfahren gemäß der vorliegenden Erfindung;
- Fig. 10: ein Flussdiagramm eines Verfahrens zur Behandlung oder Verarbeitung von Behältern gemäß der vorliegenden Erfindung;
- Fig. 11: in einer schematischen Draufsicht die Anwendung eines Verfahrens gemäß der vorliegenden Erfindung zur Gefriertrocknung einer Substanz in den Behältern;
- Fig. 12: in einem vergrößerten Teilschnitt die Anordnung der Behälter auf einem Kühlboden eines Gefriertrockenschranks bei einem Verfahren gemäß der Fig. 11;
- Fig. 13a-13g: Einzelheiten einer weiteren Haltestruktur gemäß einer weiteren Ausführungsform zur Verwendung bei einem Verfahren gemäß der vorliegenden Erfindung;
- Fig. 14a-14i: Einzelheiten einer weiteren Haltestruktur gemäß einer weiteren Ausführungsform zur Verwendung bei einem Verfahren gemäß der vorliegenden Erfindung; und
- Fig. 15a-15e: Einzelheiten einer weiteren Haltestruktur gemäß einer weiteren Ausführungsform zur Verwendung bei einem Verfahren gemäß der vorliegenden Erfindung.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur (Träger, im Stand der Technik häufig auch als sog. "Nest" bezeichnet) sowie ein Transport- und Verpackungsbehälter (im Stand der Technik häufig auch als "Tub" bezeichnet), der eine solche Haltestruktur aufnimmt, dienen gemäß der vorliegenden Erfindung, wie nachfolgend beschrieben, der gleichzeitigen Halterung einer Mehrzahl von Behältern zur Aufbewahrung von Substanzen für kosmetische, medizinische oder pharmazeutische Anwendungen in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder in regelmäßigen Reihen, die relativ zueinander versetzt angeordnet sind.

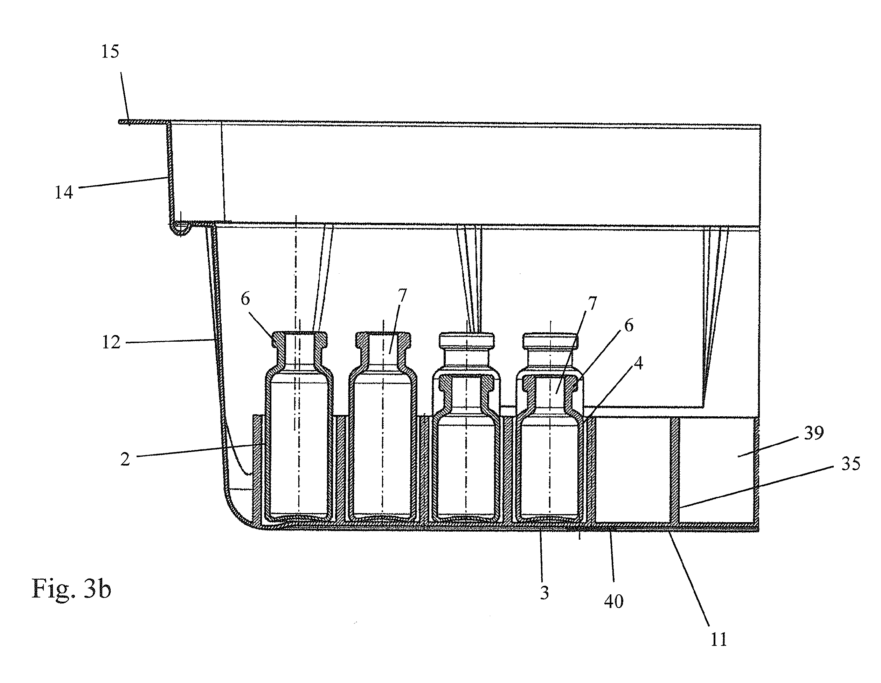

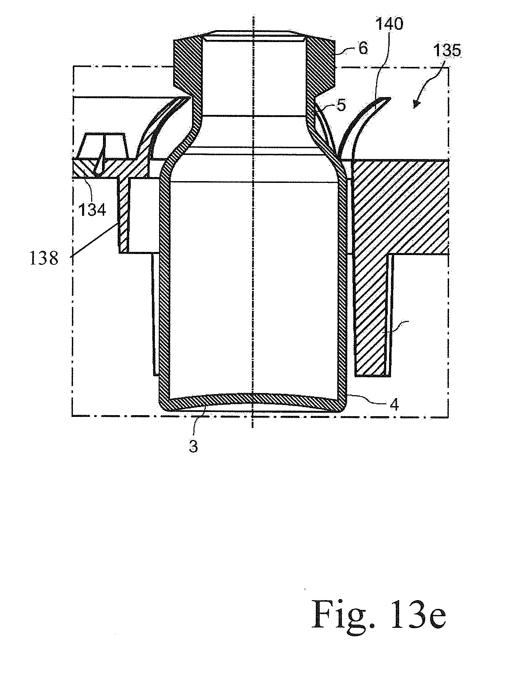

Ein Beispiel für derartige Medikamentenbehälter in Gestalt von Fläschchen (Englisch: vial) ist in der Fig. 3b oder Fig. 13e schematisch in einem Längsschnitt dargestellt und diese weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt, mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Sollbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummioder Kunststoffstopfen verschlossen ist. Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei daraufhingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Glasbehälter mittels eines Formschlusses oder eines Reibschlusses an einer Halterungsstruktur bzw. Träger fixiert. Dieser Form- oder Reibschluss kann im Bereich des verengten Halsabschnitts 5 der Behälter, an dem zylindrischen Seitenwandabschnitt 4 oder im Bereich des unteren Endes der Behälter 2, insbesondere am Boden 3 der Behälter 2 realisiert werden.

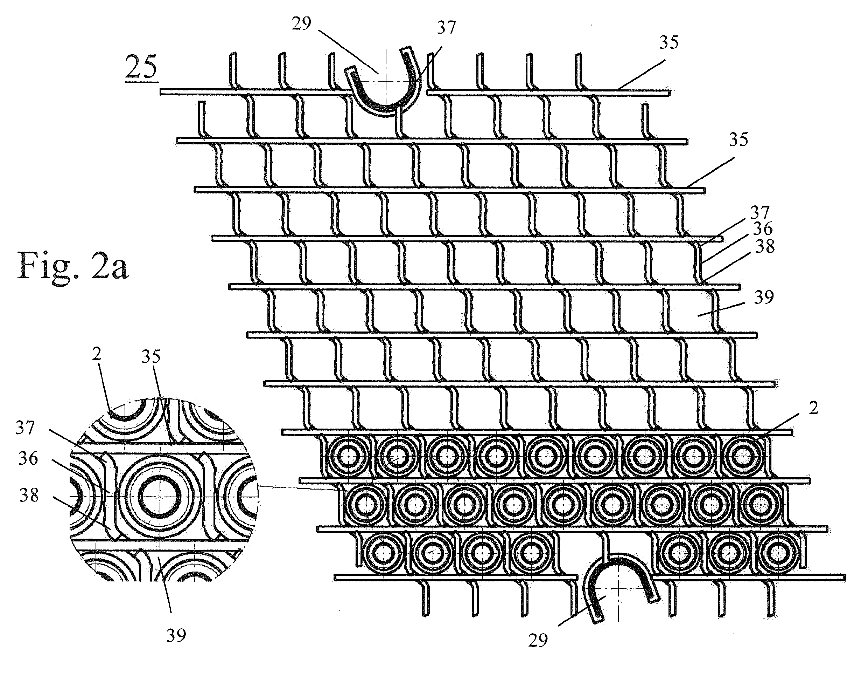

Die Fig. 2a zeigt eine Haltestruktur (Träger) 25 gemäß einem ersten Ausführungsbeispiel, mit reibschlüssiger Halterung der Behälter. Die Haltestruktur weist eine Mehrzahl von parallel zueinander verlaufenden Querstegen 35 auf, die über unter regelmäßigen Abständen zueinander angeordnete S-förmige Verbindungsstege 36 miteinander verbunden sind, die im Wesentlichen rechtwinklig zu den Querstegen 35 verlaufen. Genauer gesagt sind die Verbindungsstege 36 über in entgegengesetzte Richtungen gekrümmte vordere bzw. hintere Enden 37, 38 mit den Querstegen 35 verbunden. Die Verbindungsstege 36 sind aus einem Kunststoff hergestellt, bevorzugt aus einem flexiblen Kunststoff. Die Querstege 35 weisen bevorzugt eine größere Steifigkeit als die Verbindungsstege 36 auf. Aufgrund der S-förmigen Formgebung der Verbindungsstege 36 sind die Querstege 35 jeweils zueinander in deren Längsrichtung um eine konstante Distanz versetzt, so dass die Haltestruktur 35 insgesamt als Parallelogramm ausgebildet ist, mit einer Basis im Bereich des unteren Rands der in der Fig. 2a dargestellten Haltestruktur 25 und zwei dazu unter einem spitzen Winkel geneigt verlaufenden imaginären Randlinien, welche die vorderen Enden der Querstege 35 miteinander verbinden. In der im rechten Bildteil der Fig. 2a dargestellten entspannten Ausgangsposition können die Behälter 2 frei und ohne Berührung mit den Stegen 35, 36 oder jedenfalls mit nur geringem Kraftaufwand in die von den Stegen 35, 36 ausgebildeten länglichen Halteaufnahmen 39 eingeführt werden. Die Halteaufnahmen 39 weisen im Wesentlichen einen quadratischen Querschnitt auf, der so auf den Durchmesser der Behälter 2 abgestimmt ist, dass diese darin in einer zweiten Stellung der Haltestruktur 25 mit ausreichender Kraft reibschlüssig fixiert, insbesondere geklemmt werden können.

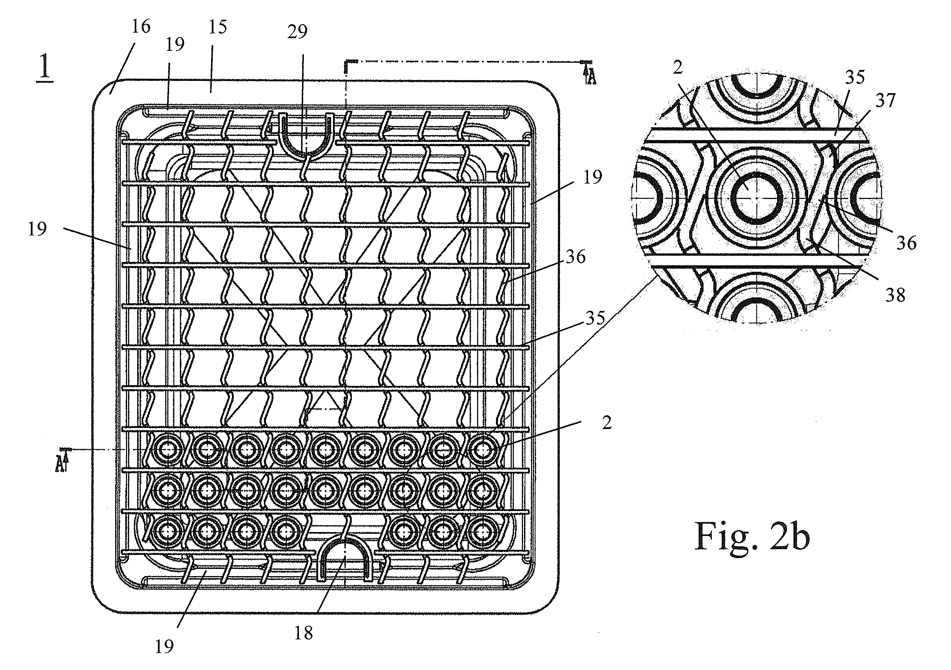

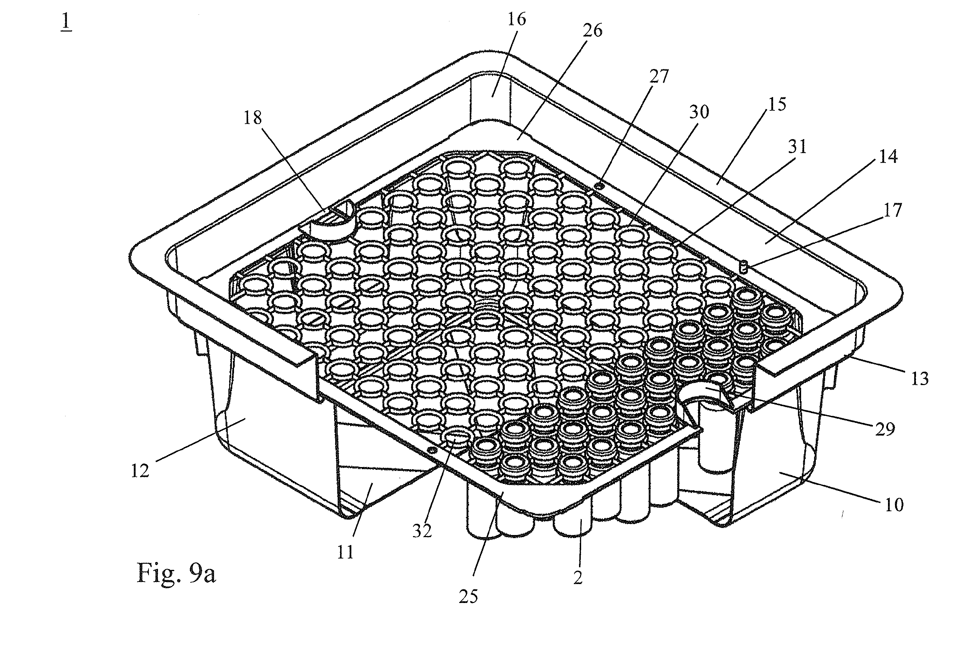

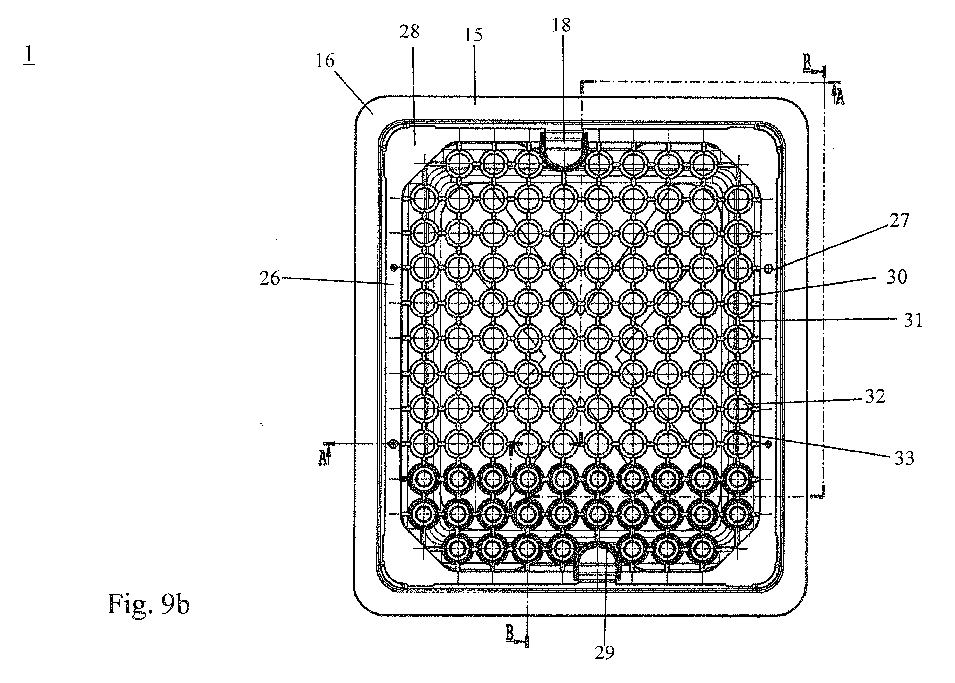

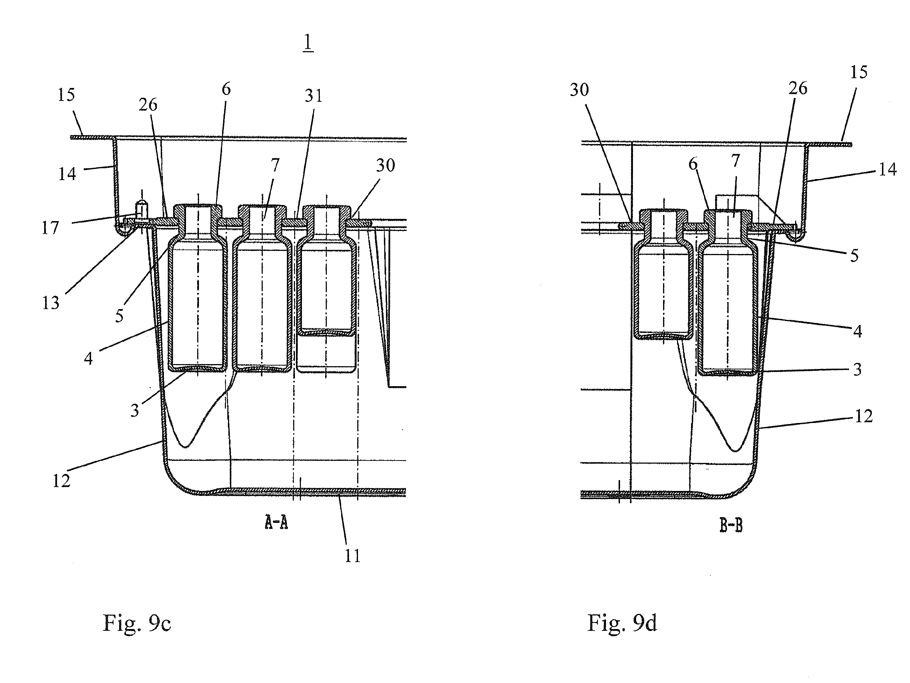

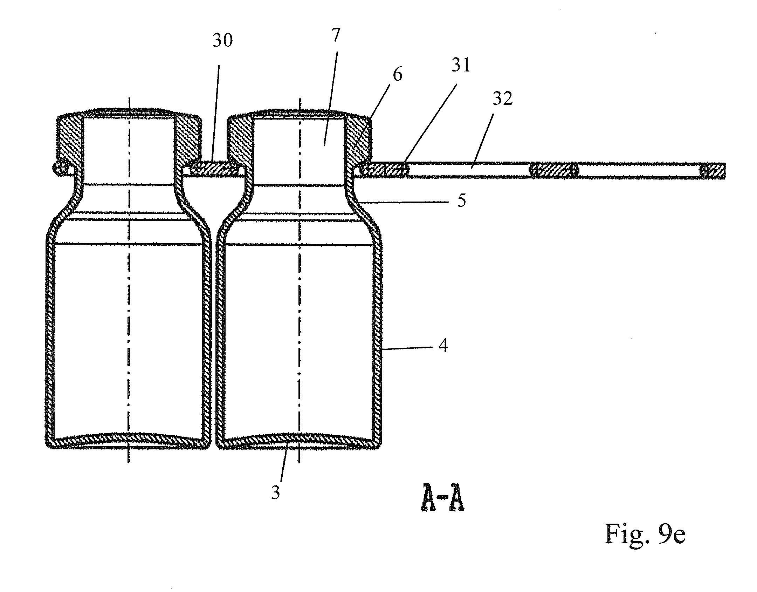

Um die Haltestruktur 25 aus der in der Fig. 2a dargestellten ersten Stellung in die in der Fig. 2b dargestellte zweite Stellung zu überführen, werden die Querstege 35 jeweils in deren Längsrichtung so verschoben, dass schließlich die in der Fig. 2b dargestellte rechteckförmige oder quadratische Haltestruktur 25 ausgebildet ist. Wie dem Vergleich der Figuren 2a und 2b entnommen werden kann, verbiegen sich hierzu die Verbindungsstege 36 geringfügig. Die zweite Stellung gemäß der Fig. 2b kann durch das Zusammenwirken der in der Haltestruktur 25 ausgebildeten Zugriffsöffnungen 29 mit korrespondierend ausgebildeten Gegenelementen des Behälters 10 fixiert werden oder auch durch das Zusammenwirken von Zentrieröffnungen 27 (vgl. Fig. 9a) mit korrespondierend ausgebildeten Zentrierzapfen 17 eines nicht dargestellten Halterahmens, welcher die Haltestruktur 25 aufnimmt.

Der vergrößerten Teildarstellung im unteren linken Teil der Fig. 2a kann man entnehmen, dass die Behälter 2 in der ersten Stellung gemäß der Fig. 2a lose in den Halteaufnahmen 39 aufgenommen sind. Der vergrößerten Teildarstellung im rechten oberen Teil der Fig. 2b kann man entnehmen, dass die Behälter 2 in der zweiten Stellung gemäß der Fig. 2b von zentralen Abschnitten der Verbindungsstege 36 reibschlüssig fixiert, insbesondere geklemmt, werden. Zu den Querstegen 35 kann weiterhin ein gewisses Spiel bestehen, das jedoch bevorzugt minimal oder verschwindend ist

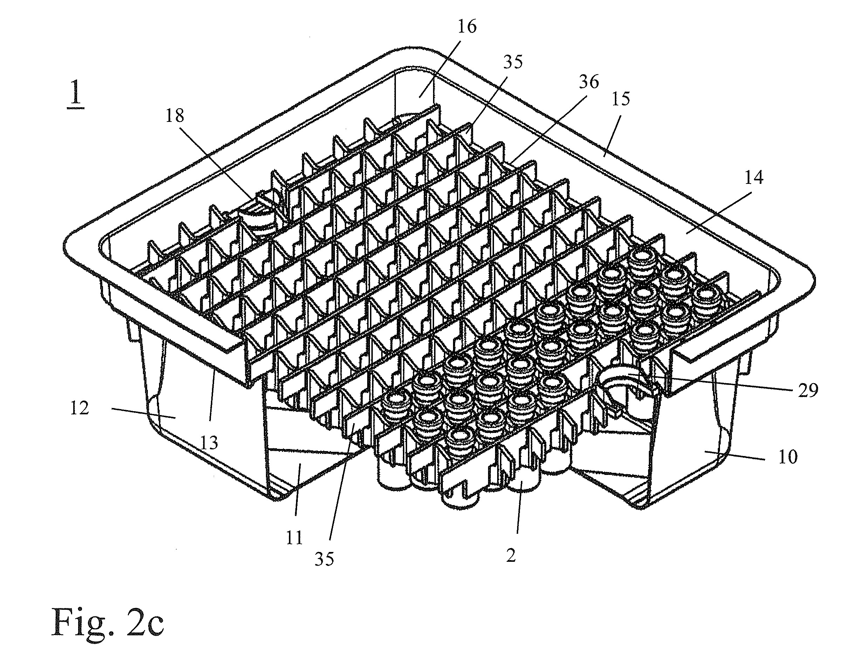

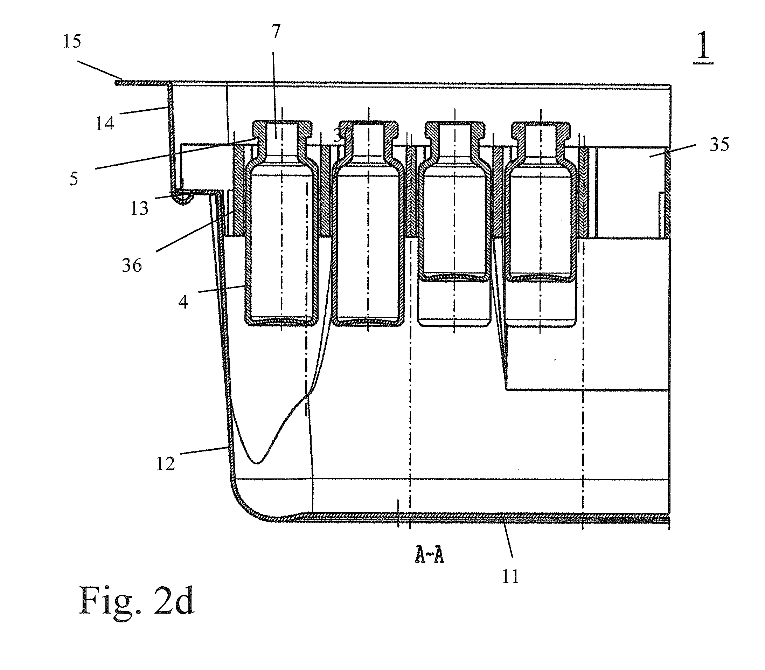

Die Fig. 2c zeigt die so ausgebildete Verpackungseinheit 1 in einem perspektivischen Teilschnitt, wobei erkennbar ist, dass bei dieser Ausführungsform die Querstege 35 unmittelbar auf der von der Stufe 13 des Behälters 10 ausgebildeten Abstützfläche abgestützt sind. Die reibschlüssige Fixierung der Behälter ist in dem Teilschnitt gemäß der Fig. 2d erkennbar.

Wie vorstehend ausgeführt, werden bei der zweiten Ausführungsform sämtliche Seitenwände der Aufnahmen 39 beim Verschieben der Querstege 35 koordiniert von der ersten Stellung in die zweite Stellung verstellt, und zwar durch Verschwenken des oberen Endes der Haltestruktur 25 (vgl. Fig. 2a) relativ zu der Basis am unteren Ende des in der Fig. 2a dargestellten Parallelogramms. In der vorgenannten ersten Stellung können die Behälter somit in die Haltestruktur eingeführt und präzise positioniert werden, insbesondere auf einem vorbestimmten Höhenniveau so angeordnet werden, dass sämtliche Böden der Behälter bündig und in einer gemeinsamen Ebene angeordnet sind. Durch koordiniertes Verstellen der Haltestruktur in die vorgenannte zweite Stellung werden dann sämtliche Behälter gleichzeitig reibschlüssig fixiert und präzise in einer regelmäßigen Anordnung positioniert. Die Haltestruktur gemäß den Figuren 2a bis 2c ist bevorzugt einstückig aus einem Kunststoff ausgebildet. Eine ähnlich wirkende Haltestruktur kann jedoch auch aus einer Mehrzahl identischer Grundeinheiten zusammengesteckt werden, wie nachfolgend beschrieben.

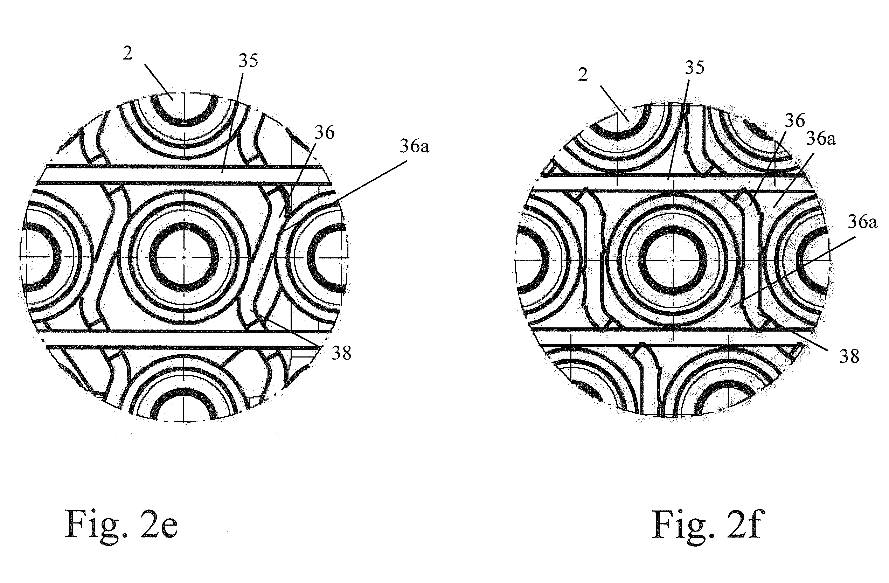

Die Figuren 2e und 2f zeigen in stark vergrößerten Teildarstellungen eine weitere Variante der Haltestruktur gemäß den Figuren 2a-2c. Dabei zeigt die Fig. 2e den Bereich einer Aufnahme der Haltestruktur in der vorgenannten zweiten Stellung, in der die Behälter 2 in den Aufnahmen der Haltestruktur gehalten sind. Abweichend zu den Figuren 2a-2c ist bei dieser Ausführungsform an den Verbindungsstegen 36 auf den beiden Seiten jeweils ein konkav ausgebildeter Abschnitt 36a ausgebildet, wobei der Krümmungsradius der beiden konkaven Aufnahmen der Abschnitte 36a auf den Radius der Behälter 2 abgestimmt ist. In der zweiten Stellung gemäß der Fig. 2e, in welcher die Verbindungsstege 36 sich geneigt zu den Querstegen 35 erstrecken, schmiegen sich die konkaven Aufnahmen 36a an die zylindrische Seitenwand der Behälter 2 an, sodass die Behälter noch zuverlässiger und kontrollierter gehalten werden können. In der ersten Stellung gemäß der Fig. 2f, in welcher die Verbindungsstege 36 sich senkrecht zu den Querstegen 35 erstrecken, sind die konkaven Aufnahmen 36a nicht mehr gegenüberliegend zur zylindrischen Seitenwand der Behälter 2 angeordnet, sodass die Behälter ungehindert, jedenfalls mit deutlich reduziertem Kraftaufwand, in die von den Stegen 35, 36 ausgebildeten Aufnahmen eingeführt und aus diesen herausgenommen werden können. Idealerweise liegen die Stege 35, 36 in der ersten Stellung gemäß der Fig. 2f überhaupt nicht an den Seitenwänden der Behälter 2 an.

Mit Hilfe eines Trägers gemäß der Fig. 2a oder wie nachfolgend beschrieben, können somit eine Mehrzahl von Behältern gleichzeitig oder chargenweise behandelt oder verarbeitet werden, während diese an einem Träger gehalten oder jedenfalls geführt sind. Gemäß weiteren Ausführungsformen der Erfindung kann eine solche Behandlung oder Verarbeitung auch dann erfolgen, während der Träger mit den von diesem gehaltenen Behältern in einem Transport- und Verpackungsbehälter angeordnet ist, wie dieser beispielhaft in der Fig. 2c dargestellt ist. Hierzu kann der Träger 25 (vgl. Fig. 2c) beispielsweise über die Zugriffsöffnungen 29 geeignet angehoben werden, ohne jedoch aus dem Transport- und Verpackungsbehälter 1 entnommen zu werden. Geht trotz aller Vorsichtsmaßnahmen einmal ein Behälter zu Bruch oder kommt es in anderer Weise während der Behandlung oder Verarbeitung zu Verunreinigungen, so werden diese Verunreinigungen jedenfalls in dem Transport- und Verpackungsbehälter zurückgehalten, geraten somit jedenfalls nicht in die eigentliche Prozessanlage.

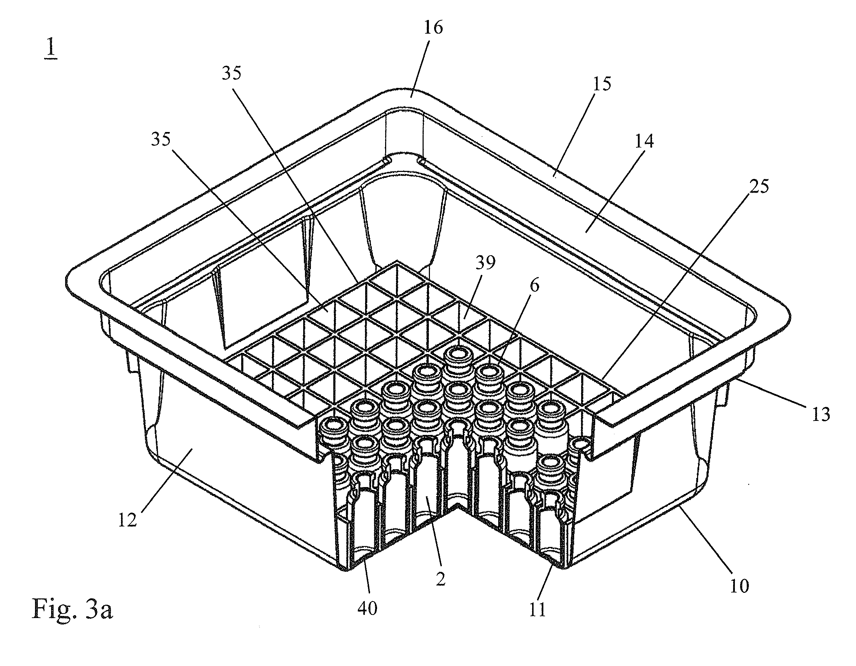

Während vorstehend dargelegt wurde, dass die Haltestruktur auf der von der Stufe 13 nahe dem oberen Rand ausgebildeten Auflagefläche abgestützt ist, ist nach dem Ausführungsbeispiel gemäß der Fig. 3a die Haltestruktur 25 unmittelbar auf dem Boden 11 des Transport- und Verpackungsbehälters 10 abgestützt. Dies funktioniert grundsätzlich bei sämtlichen Haltestrukturen, die über eine gewisse axiale Länge verfügen, sei es dass die Böden der Behälter 2 unmittelbar auf dem Boden 11 des Behälters 10 aufliegen oder auf Böden der länglichen Aufnahmen 39 der Haltestruktur 25 aufliegen. Bei dem dargestellten Ausführungsbeispiel gemäß der Fig. 3a ist die Haltestruktur 25 durch eine Mehrzahl von sich rechtwinklig kreuzenden Querstegen 35 ausgebildet, die eine Mehrzahl von länglichen Aufnahmen 39 mit quadratischem Querschnitt ausbilden, wobei die Aufnahmen 39 in einer Matrix-Anordnung angeordnet sind. Dabei werden die Behälter 2 an ihrem unteren Ende reibschlüssig von den Querstegen 35 oder einer darauf vorgesehenen Einlage aus einem flexiblem Kunststoff fixiert. Die Fig. 3b zeigt einen schematischen Teilschnitt durch diese Haltestruktur. Dargestellt ist beispielhaft die Klemmung von Behältern 2 mit unterschiedlicher Höhe. Grundsätzlich kann die Haltestruktur 25 auch einstückig mit dem Boden 11 des Behälters 10 ausgebildet sein.

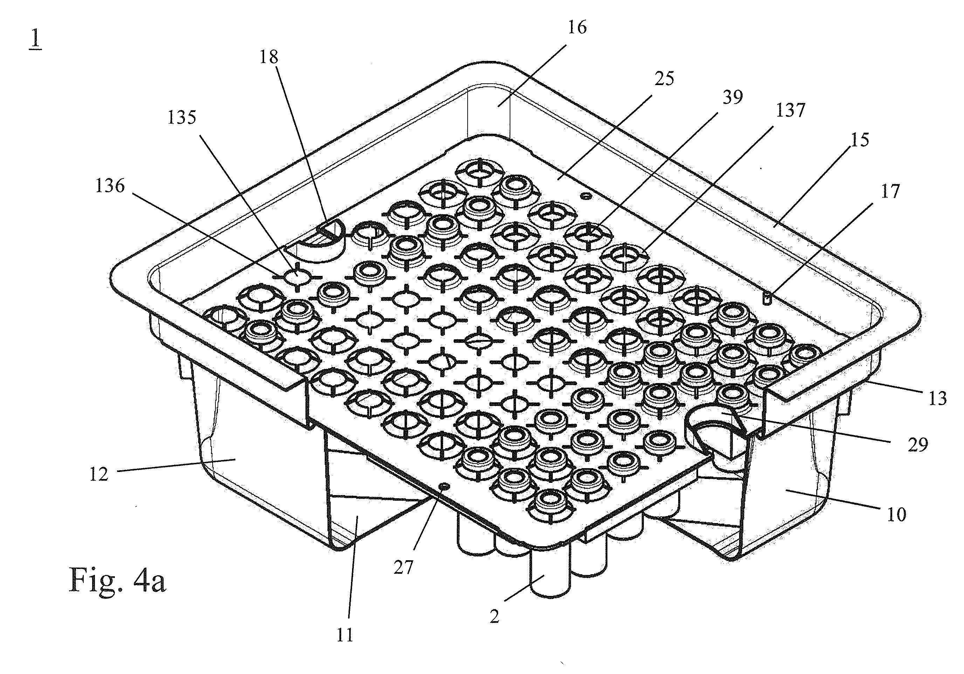

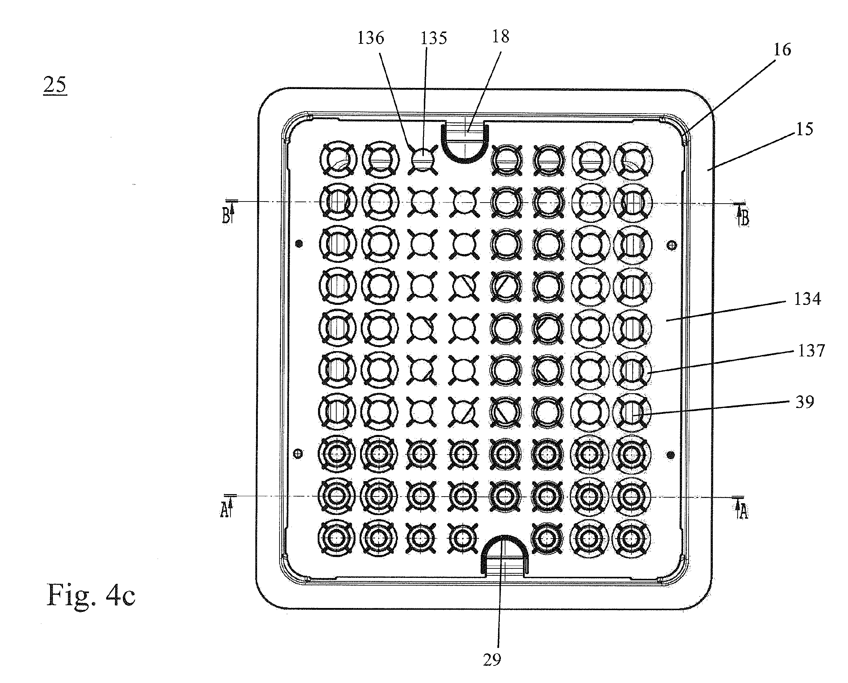

Die Figuren 4a bis 4c zeigen ein weiteres Ausführungsbeispiel für eine Haltestruktur (Träger), woran eine Mehrzahl von Behältern 2 formschlüssig gehalten werden können. Hier sind gemäß der Fig. 4a in der flächigen Transportplatte 25 eine Mehrzahl von Öffnungen 39 zur Aufnahme der Behälter 2 ausgebildet.

Die Öffnungen sind in ringförmigen Formschlusselementen 137 ausgebildet, die entweder in die Öffnungen 39 eingesteckt sind, insbesondere in deren Umfangsrand eingerastet oder eingeclipst sind, oder die einstückig mit dem flächigen Träger 25 ausgebildet sind, beispielsweise durch ein 1K- oder 2K-Kunststoff-Spritzgussverfahren.

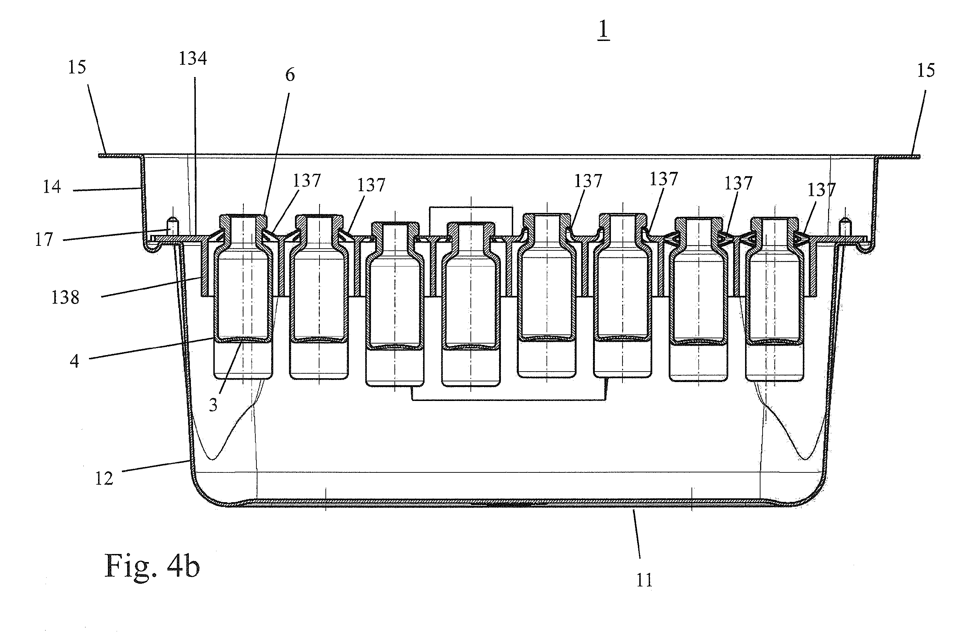

Der schematische Längsschnitt gemäß der Fig. 4b stellt summarisch mehrere verschiedene Ausführungsvarianten für Formschlusselemente 137 in einer vergleichenden Darstellung dar. Im fixierten Zustand stützen die Formschlusselemente 137 die Behälter unmittelbar unterhalb des oberen Rands 6 und im Bereich des verengten Halsabschnitts 5 ab.

Wenngleich in der Fig. 4a der Boden 11 des Behälters 10 als geschlossen und einstückig mit der Seitenwand 12 ausgebildet dargestellt ist, kann das untere Ende des Behälters 10 auch in der Art des oberen Endes geöffnet ausgebildet sein, insbesondere mit einem flanschartigen unteren Rand in der Art des oberen Rands 15 versehen sein, so dass die Böden der Behälter 2 von der Unterseite des Behälters 10 her frei zugänglich sind, beispielsweise für Verarbeitungsschritte in einem Steriltunnel oder in einem Gefriertrockenschrank, wie nachfolgend näher erläutert.

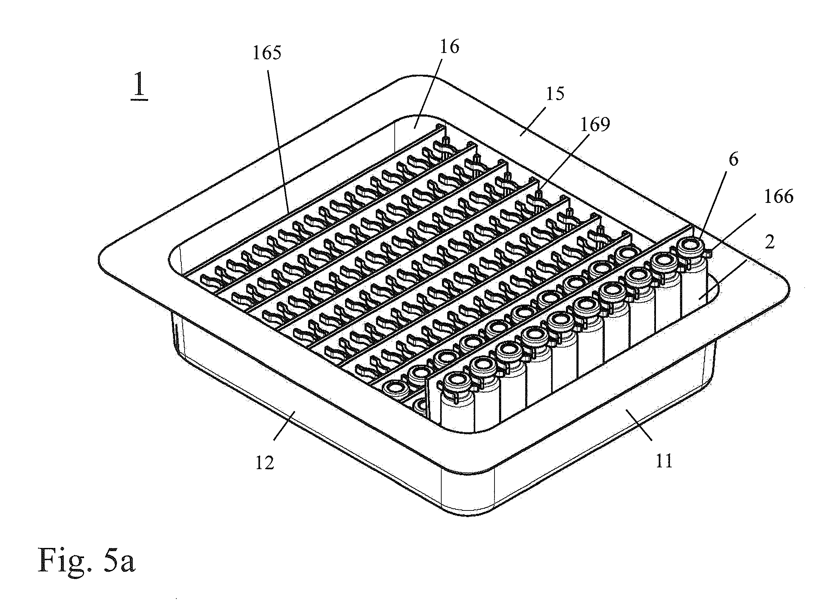

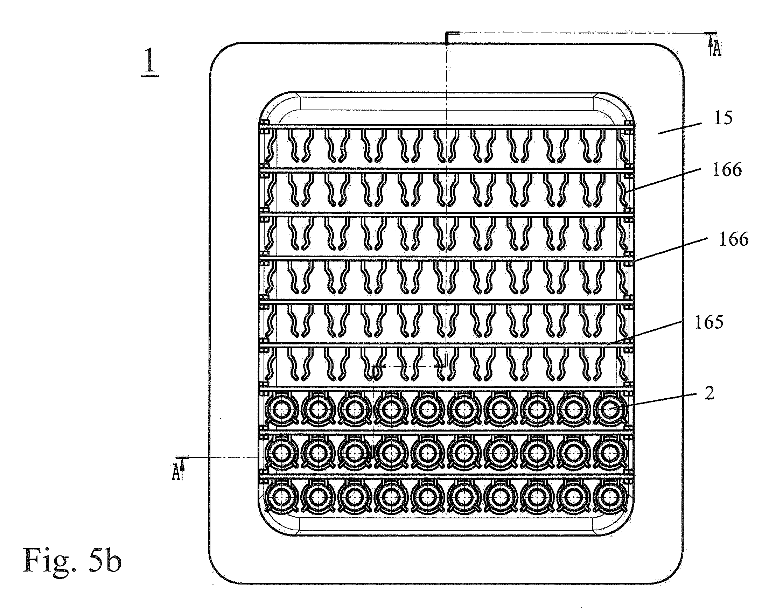

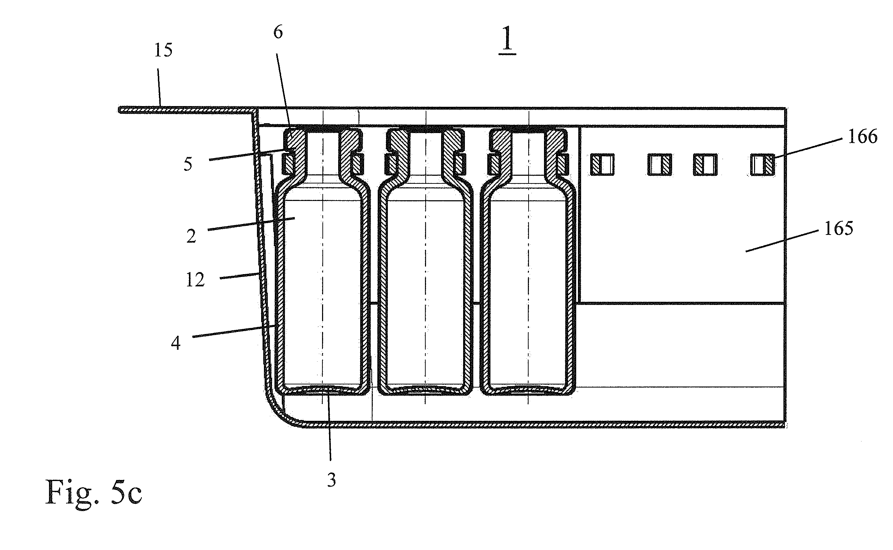

Die Figuren 5a bis 5c zeigen eine weitere Ausführungsform eines Transport- und Verpackungsbehälters 1. Gemäß der Fig. 5a ist die Haltestruktur 25 als Kasten ausgebildet, mit umlaufenden Seitenwänden 168. Dieser Kasten 168 wird von einer Mehrzahl von sich parallel erstreckenden und zueinander unter regelmäßigen Abständen zueinander beabstandeten Querstegen 165 in mehrere rechteckförmige Segmente unterteilt. Auf den Oberflächen der Querstege 165 sind auf gleicher Höhe und unter regelmäßigen Abständen zueinander konkav gebogene Haltearme 166 angeordnet, die aus einem elastischen Kunststoff ausgebildet sind und entweder einstückig mit den Querstegen 165 ausgebildet sind oder an diesen befestigt oder angeformt sind. Die Haltearme 166 bilden Aufnahmen, in welche die Behälter so von vorne her eingeführt werden können, dass deren Halsabschnitte formschlüssig umgriffen und der obere Rändern auf diesen abgestützt werden können, wie in der Fig. 5c dargestellt. In Längsrichtung der Behälter 2 betrachtet besteht ein Reibschluss. Durch Aufbringen einer geeigneten axial wirkenden Kraft können die Behälter 2 jedoch in den von den Haltearmen 166 ausgebildeten Aufnahmen axial verschoben werden, beispielsweise in eine angehobene Stellung.

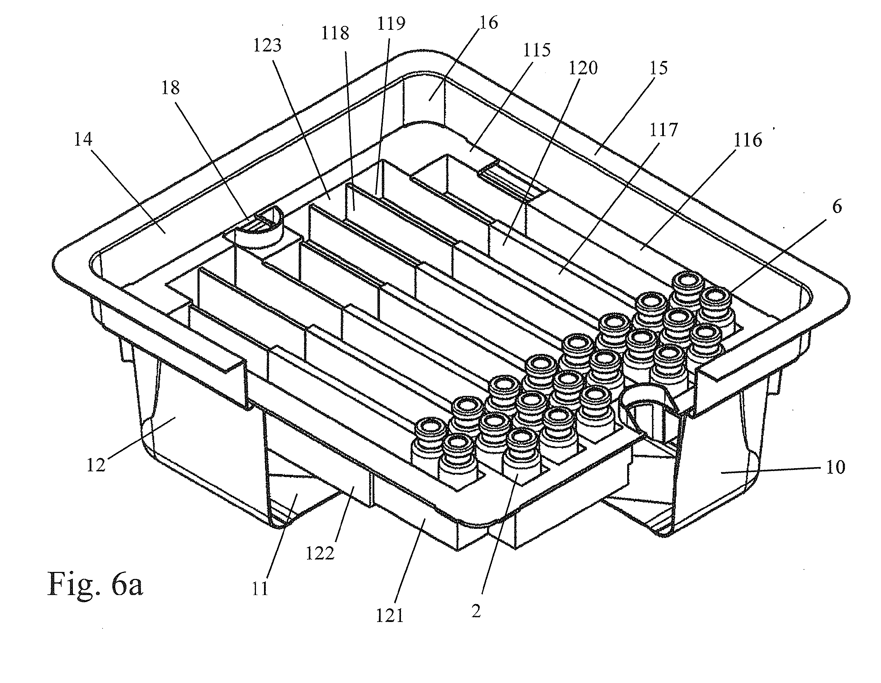

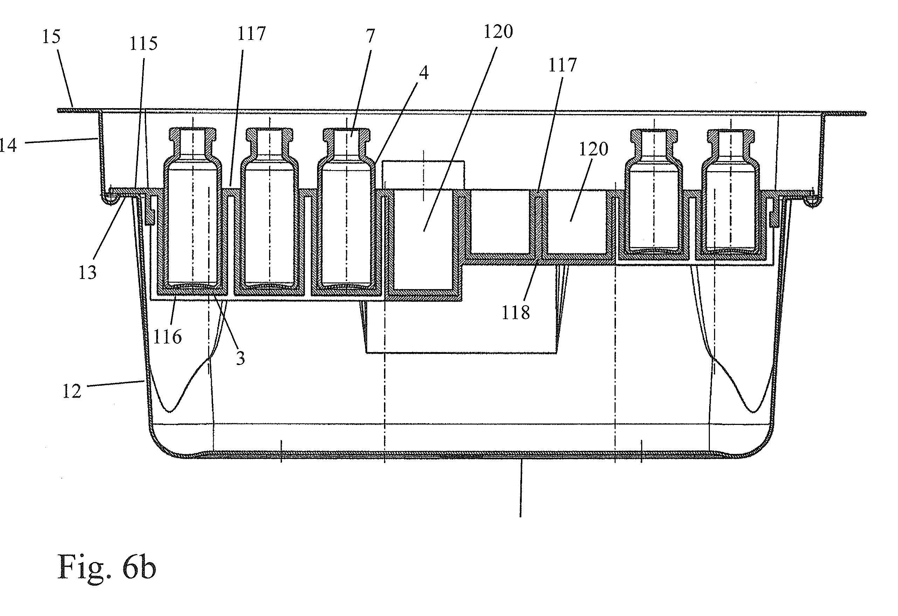

Die Fig. 6a zeigt eine weitere Ausführungsform für einen Transport- und Verpackungsbehälter 1 mit einem Halteeinsatz, der von zwei ineinander schiebbaren Teilen ausgebildet ist, die gemeinsam längliche, im Profil rechteckförmige und parallel zueinander verlaufende Halteaufnahmen 120 ausbilden, in welchen die Behälter 2 aufgenommen und reibschlüssig fixiert sind. Genauer gesagt sind in dem rechten Schiebeteil 116 von den Führungswänden 117 eine Mehrzahl von sich parallel zueinander erstreckenden rechteckförmigen Aufnahmen 120 ausgebildet und sind in dem linken Schiebeteil 115 von den Seitenwänden 118 entsprechende längliche Aufnahmen ausgebildet. Die Führungswände 117 sind geschlitzt ausgebildet, wie man der Schnittansicht gemäß der Fig. 6b entnehmen kann. In den Längsschlitzen der Führungswände 117 sind die korrespondierend ausgebildeten Seitenwände 118 des linken Schiebeteils 115 verschieblich geführt und aufgenommen. Die beiden Schiebeteile 115, 116 können ineinander geschoben werden, bis vordere bzw. hintere Enden 123 der Aufnahmen 120 unmittelbar an den in den Aufnahmen 120 aufgenommenen Behälter 2 anliegen, so dass die Behälter 2 von den Seitenwänden der Aufnahmen 120 gemeinsam geklemmt werden. Grundsätzlich können die Seitenwände der Aufnahmen 120 mit Haltevorsprüngen bzw. Vertiefungen korrespondierend zu den Behältern 2 ausgebildet sein, so dass die Behälter 2 einander nicht unmittelbar berühren, wenn diese in den Aufnahmen 120 aufgenommen sind, sondern in den von den Vorsprüngen bzw. Vertiefungen ausgebildeten Aufnahmen beabstandet zueinander aufgenommen sind.

Gemäß einer bevorzugten weiteren Ausführungsform sind die Seitenwände 118, 122 des linken Schiebeteils 115 in Längsrichtung der Aufnahmen 120 betrachtet keilförmig ausgebildet, so dass die Öffnungsweite zwischen den Seitenwänden 117 des rechten Schiebeteils 116 zunehmend verringert wird, solange bis die Behälter 2 in den länglichen Aufnahmen 120 reibschlüssig fixiert, insbesondere geklemmt sind. Die Fig. 6b ist eine schematische Schnittansicht und zeigt die Aufnahme von Behältern mit unterschiedlichen Höhen in Aufnahmen 120 mit unterschiedlichen Höhen (linker und rechter Bildteil).

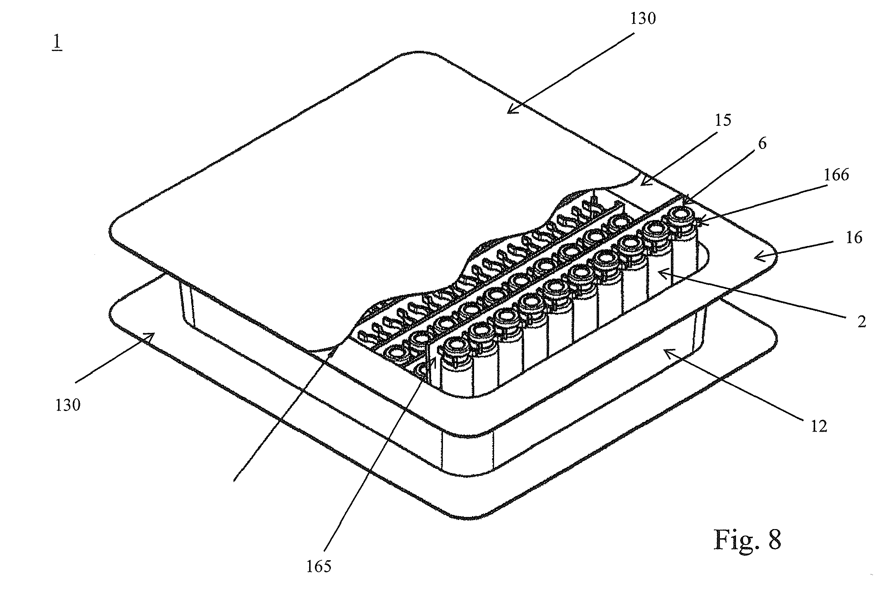

Die Oberseite oder die Ober- und Unterseite einer Haltestruktur 25 gemäß der vorliegenden Erfindung oder auch eines Transport- und Verpackungsbehälters 1 gemäß der vorliegenden Erfindung kann bzw. können von einer sterilen, gasdurchlässigen Schutzfolie überzogen sein, die aufgeklebt und bei Bedarf abziehbar ist. Dies ist beispielhaft in der Fig. 8 für eine Verpackungseinheit dargestellt, die von einem beidseitig offenen Transportbehälter und einer darin aufgenommen Haltestruktur gemäß der Fig. 5a ausgebildet ist und auf der Ober- und Unterseite mittels einer auf den Rand 15 aufgeklebten Schutz- oder Verpackungsfolie 130 verschlossen ist. Bei der Schutzfolie 130 kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Behälter 2 durch die Folie 130 hindurch ermöglicht.

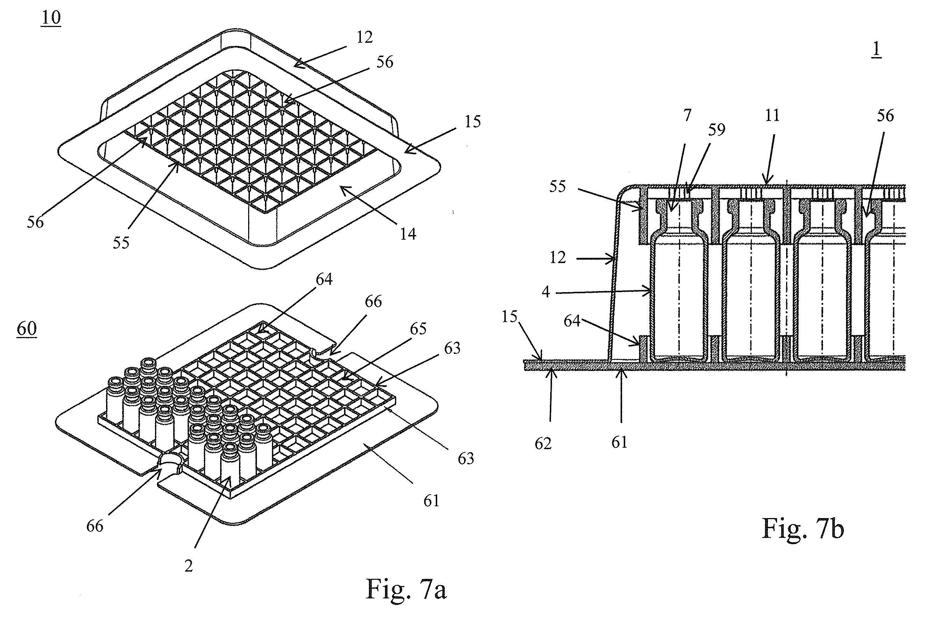

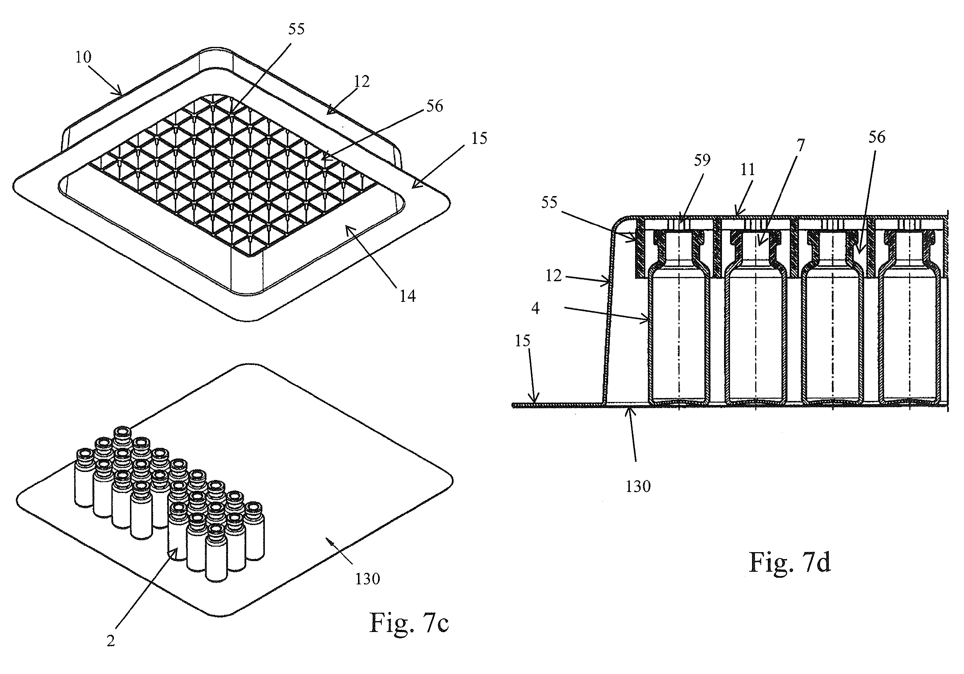

Bei einer weiteren Ausführungsform gemäß der Fig. 7a sind die Behälter 2 unmittelbar auf einer Schutz- oder Verpackungsfolie 130 angeordnet, auf die ein Transport- und Verpackungsbehälter 10, wie vorstehend anhand der Fig. 2a beispielhaft beschrieben, aufgesetzt wird, wobei die auf dem Boden des Behälters 10 angeordneten Querstege 55 eine unmittelbare Berührung der Seitenwände 4 der Behälter verhindern. An ihrem oberen Ende können die Behälter durch die Querstege 55 auch reibschlüssig fixiert werden, insbesondere geklemmt werden. Bei der Folie 130 kann es sich insbesondere um eine sterile, jedoch gasdurchlässige Folie handeln, insbesondere um ein Kunststoff-Geflecht, wie beispielsweise Tyveck^{®}.

Die Figuren 7c bis 7e zeigen eine weitere Variante zu dieser Ausführungsform, bei der die in dem Transport- und Verpackungsbehälter 10 aufgenommenen Behälter durch Einblasen eines Gases durch die Folie 130 hindurch sterilisiert werden. Damit einströmende Gase in den Innenraum der Behälter 2 einströmen können, sind zwischen dem Boden 11 des Transport- und Verpackungsbehälters 10 und dem oberen Rand der Behälter 2 Abstandshalter 59 vorgesehen, sodass die Behälter nicht unmittelbar auf dem Boden 11 aufliegen.

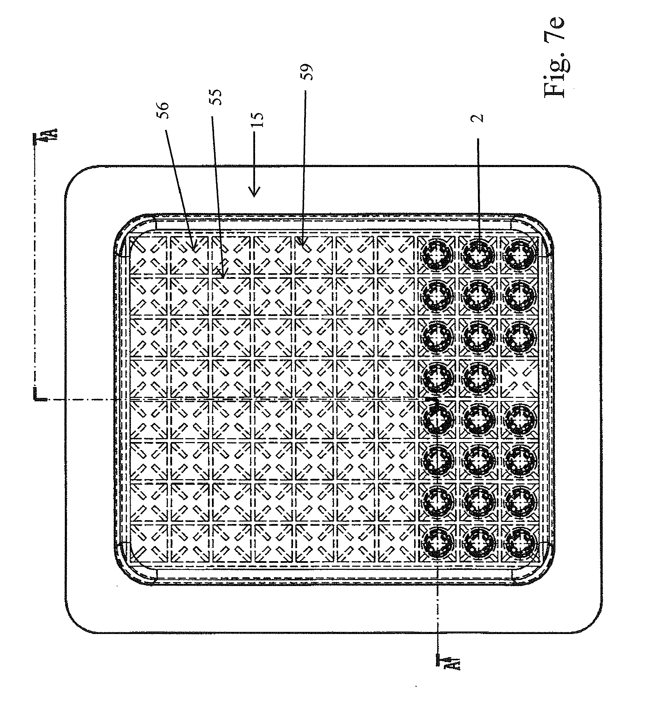

Diese Abstandshalter 59 können sich ausgehend von den Ecken einer jeweiligen Aufnahme 56 diagonal zur Mitte der jeweiligen Aufnahme 56 hin erstrecken. Die kreuzförmigen Abstandshaltestege 59 sind jedoch nicht miteinander verbunden, sodass im Bereich der Mitte einer jeweiligen Aufnahme 56 der obere Rand der Behälter ungehindert zugänglich ist. Die Fig. 7e zeigt eine Draufsicht auf den auf dem Boden des Transport- und Verpackungsbehälters 10 ausgebildeten Einsatz, der auch herausnehmbar sein kann.

Gemäß einer weiteren Ausführungsform gemäß den Figuren 9a bis 9e wird zum gleichzeitigen Halten einer Mehrzahl von Behältern 2 eine flächige Transportplatte 25 bereitgestellt, die aus einem Kunststoff ausgebildet ist, beispielsweise ausgestanzt oder spritzgegossen ist, und die aus einer Mehrzahl von kreisförmigen Ringösen 30 besteht, die miteinander verbunden sind. Die Ringösen 30 sind ausreichend flexibel oder aufweitbar, so dass die Behälter 2 von oben oder von unten her in die Öffnungen der Ringösen 30 eingeführt werden können. Dadurch können eine Mehrzahl von Behältern 2 im Bereich ihrer verengten Halsabschnitte 5 reibschlüssig fixiert werden. Dies ist in den schematischen Längsschnitten gemäß den Figuren 9c bis 9e genauer dargestellt.

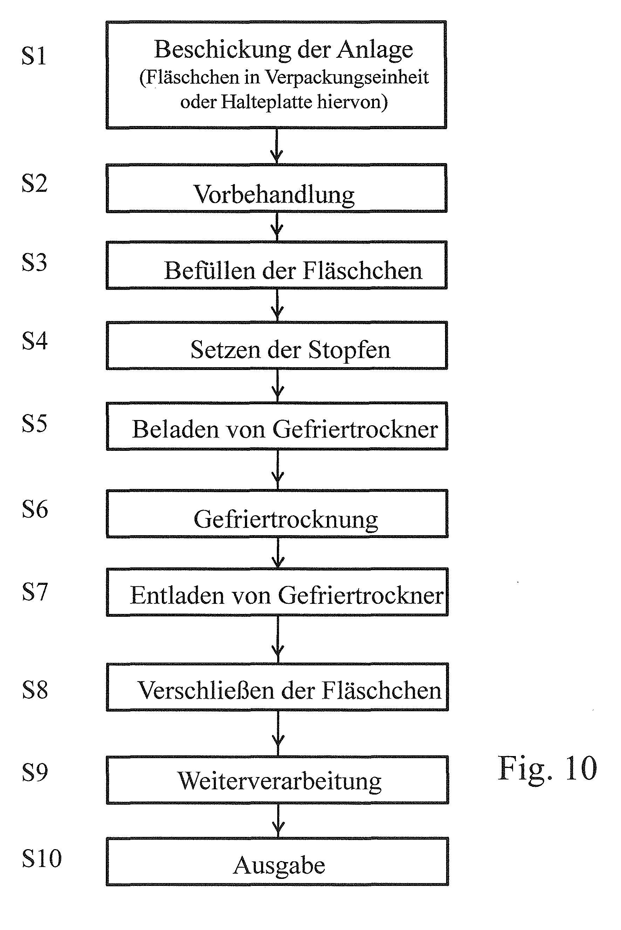

Bei den meisten vor vorstehend beschriebenen Ausführungsbeispielen sind die Böden der Behälter von der Unterseite her vollständig zugänglich, wenn diese gemeinsam an dem Träger gehalten werden. Dies ermöglicht beispielsweise die chargenweise Gefriertrocknung einer Mehrzahl von Behältern in einem Gefriertrockenschrank, während diese gemeinsam an dem Träger gehalten sind. Dies wird nachfolgend anhand der Figuren 10 bis 12 näher beschrieben werden.

Die Fig. 10 zeigt ein schematisches Flussdiagramm eines solchen Prozessschrittes, wobei im Unterschied zu einem herkömmlichen Prozessschritt, wie vorstehend anhand der Fig. 1 beschrieben, eine Mehrzahl von Behältern in den Prozessschritten S1 bis S9, jedenfalls in den Prozessschritten S5 bis S7, gemeinsam an einem Träger gehalten oder zumindest von diesem geführt aufgenommen sind.

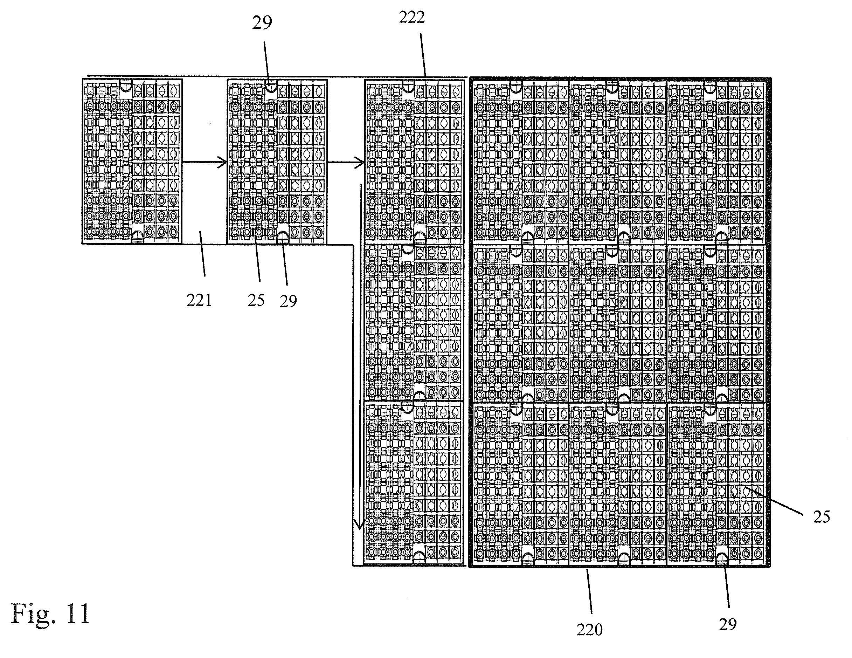

Dies ist schematisch in der Draufsicht gemäß der Fig. 11 dargestellt. Die Träger 25 mit den von diesen in einer regelmäßigen zweidimensionalen Anordnung gehaltenen Behältern werden mittels der Fördereinrichtung 221, beispielsweise eines Transportbands oder einer Rollenbahn, in Pfeilrichtung in Richtung zu einem Gefriertrockenschrank 220 gefördert. Dieser kann beispielsweise seitlich zu einer Haupt-Fördereinrichtung einer nicht dargestellten Prozessanlage angeordnet sein, von der die Träger 25 auf die Fördereinrichtung 221 umgesetzt bzw. umgelenkt und in Richtung zu einem Gefriertrockenschrank 220 gefördert werden. Vor dem Gefriertrockenschrank 220 befindet sich eine quer zur Fördereinrichtung 221 verlaufende Ablagefläche, auf der die Träger gesammelt werden. Diese Sammlung der Träger 25 vor dem Gefriertrockenschrank 220 kann auch auf mehreren Ebenen erfolgen, in Entsprechung zu den Ebenen des Gefrieriroclcenschranks 220.

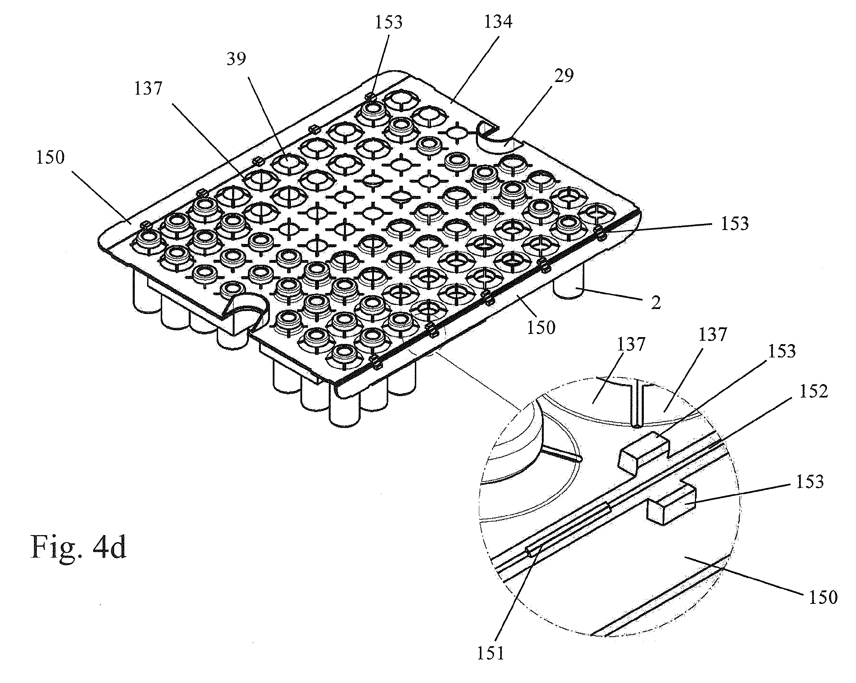

Um die Grundfläche der Träger 25 weiter zu reduzieren, kann es hierfür von Vorteil sein, wenn Randabschnitte 150 der Träger (vgl. Fig. 4d) abgenommen oder, wie in der Fig. 4d dargestellt, weggeschwenkt werden können. Zu diesem Zweck sind die Ränder 150 über Scharniere 151 mit dem Träger 134 gemäß der Fig. 4d verbunden. Auf der Oberseite des Trägers 134 und der Ränder 150 sind an einander entsprechenden Positionen blockförmige Anschläge 153 vorgesehen, die im gegenseitigen Anschlag eine koplanare Ausrichtung der Ränder 150 und des Trägers 134 festlegen. Gemäß einer weiteren Ausführungsform (nicht dargestellt) können die Ränder 150 auch von dem Träger 134 abgenommen werden. Die Ränder 150 können selbstverständlich entlang allen vier Längsseiten des Trägers 134 vorgesehen sein.

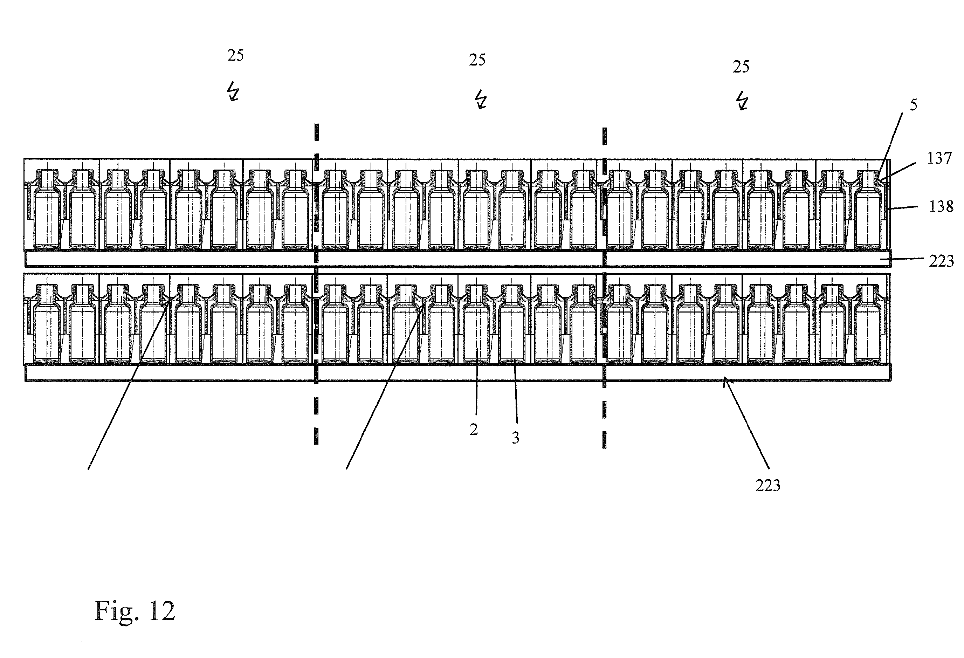

Diese einfache Maßnahme erhöht die erzielbare Packungsdichte der Behälter 2 beim Beladen des Gefriertrockenschranks 220 (vgl. Fig. 11) weiter. Die Fig. 12 zeigt einen vergrößerten Teilschnitt durch einen Gefriertrockenschrank. Wie man erkennen kann, liegen die Böden 3 der Behälter 2 unmittelbar auf den Kühlböden 223 auf, sodass ein optimaler Kühleffekt erzielt werden kann. Die Kühlböden 223 sind dabei auf mehreren Etagen angeordnet.

Ein entsprechendes Anheben und Absenken der Behälter, während diese in Trägern gehalten oder zumindest in diesen geführt sind, eignet sich vorteilhaft auch für die Inspektion der Behälter oder deren Inhalts, insbesondere mittels optischer Prüfverfahren. Wie man den meisten Darstellungen dieser Anmeldung entnehmen kann, ragen die Behälter mit ihrem oberen Rand zumeist nicht über den oberen Rand des zugeordneten Trägers hinaus, sodass diese nicht optisch geprüft und beurteilt werden können. Dies ist zumeist auch nicht zuverlässig möglich, wenn der Transport- und Verpackungsbehälter aus einem transparenten Kunststoff ausgebildet ist. In der vorstehend beschriebenen angehobenen Stellung sind die Behälter jedoch zumindest in deren oberem Bereich für ein Prüfverfahren oder zur Begutachtung zugänglich. Dies kann ausgenutzt werden, wenn man beispielsweise die Behälter in einer Fördereinrichtung mittels einer rampenartigen Führungsfläche, die in die Bahn der Fördereinrichtung geeignet eingreift, gezielt anhebt und anschließend wieder zurück in die Aufnahmen der Träger absenkt.

Ein entsprechendes Anheben und Absenken der Behälter, während diese in Trägern gehalten oder zumindest in diesen geführt sind, wie vorstehend beschrieben, eignet sich auch vorteilhaft für eine koordinierte Übergabe der Behälter an nachgeordnete Prozessstationen. Beispielsweise können die Behälter einzeln oder reihenweise in der vorstehend beschriebenen angehobenen Stellung, in welcher die Haltekraft in den Aufnahmen oder Öffnungen des Trägers vollständig oder jedenfalls in ausreichendem Maße für deren Entnahme aufgehoben ist, mit einem Greifer gegriffen werden, beispielsweise an deren oberem Rand, und koordiniert übergeben werden.

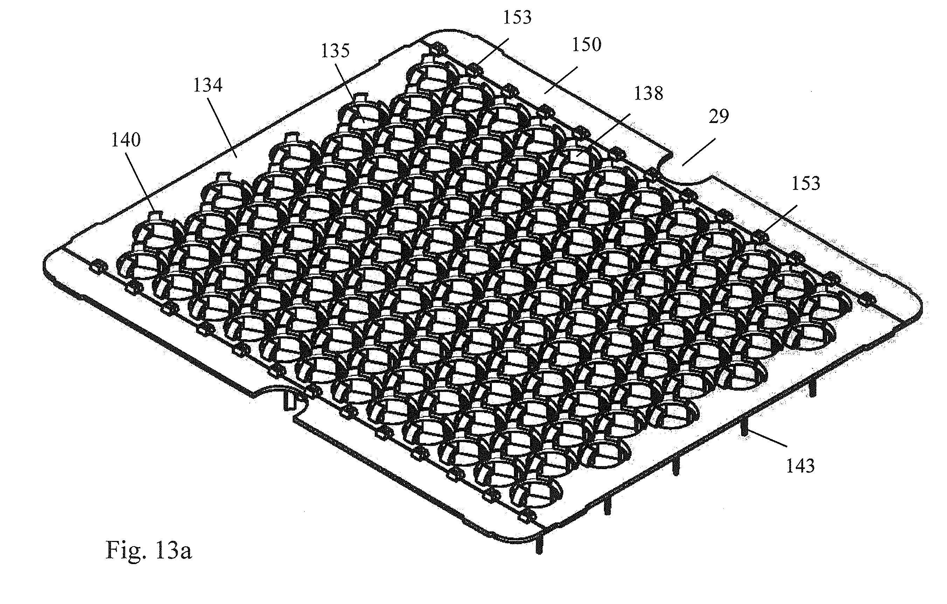

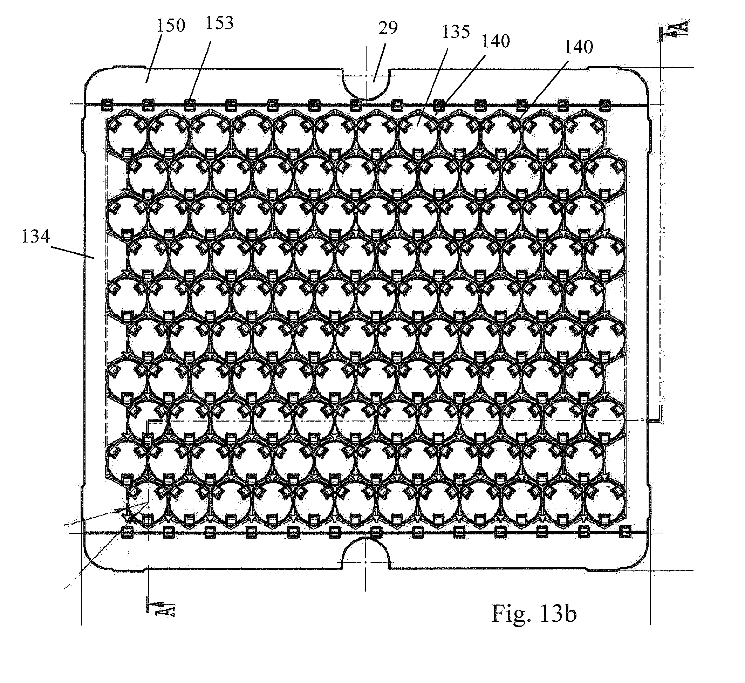

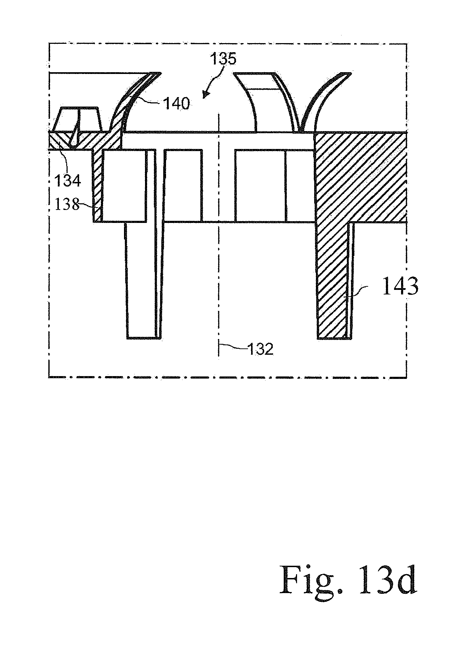

Zum gleichzeitigen Halten einer Mehrzahl von Behältern wird gemäß einer weiteren Ausführungsform der vorliegenden Erfindung, wie in den Figuren 13a und 13b dargestellt, ein flächiger rechteckförmiger Träger 134 bereitgestellt, der aus einem Kunststoff ausgebildet ist, beispielsweise ausgestanzt oder spritzgegossen ist, und eine Mehrzahl von Öffnungen 135 zur Aufnahme der Glasfläschchen 2 aufweist. Die Öffnungen 135 sind von Seitenwänden 138 (vgl. Fig. 13d) auf der Unterseite des Trägers 134 begrenzt. Gemäß der Fig. 13b ragen elastische Haltezungen 140 von der Oberseite des Trägers 134 bogenförmig ab und, in Draufsicht betrachtet, in die zugeordneten Öffnungen 135 hinein. Die elastischen Haltezungen 140 und die Seitenwände 138 sind bevorzugt einstückig mit dem flächigen Träger 134 ausgebildet, beispielsweise durch ein 1K- oder 2K-Kunststoff-Spritzgussverfahren.

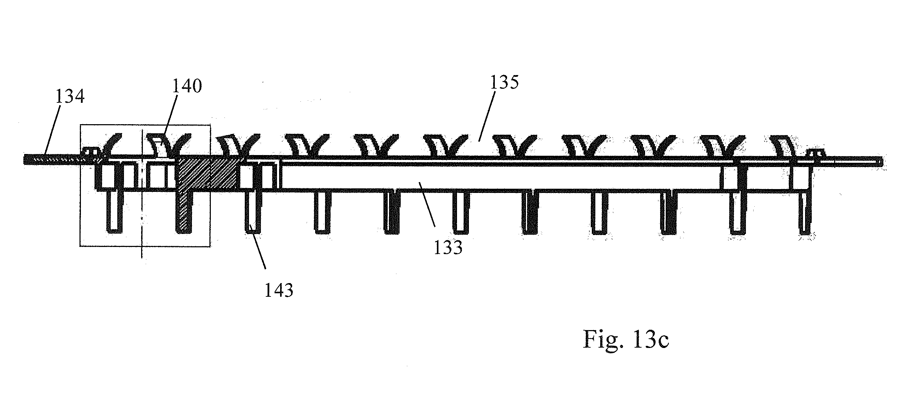

Wie man der Zusammenschau der Figuren 13b und 13d entnehmen kann, sind die Seitenwände 138 in einer regelmäßigen hexagonalen Anordnung auf der Unterseite des Trägers 134 verteilt angeordnet. Die Seitenwände 138 sind umlaufend ausgebildet, können jedoch als nur vergleichsweise kurze Seitenwandabschnitte ausgebildet sein, um eine zugeordnete Öffnung oder Aufnahme nur abschnittsweise zu begrenzen. In jedem Fall wird eine Kollision von Behältern, die in unmittelbar benachbarten Öffnungen 135 aufgenommen sind, durch die Seitenwände 138 verhindert. Gemäß der Fig. 13c ragen von der Unterseite des Trägers 134 Zapfen 143 ab, mit welchen der Träger 134 auf einer Ablagefläche und beabstandet zu dieser abgelegt werden kann.

Gemäß der Fig. 13b laufen die Seitenwände 138 jeweils in den Eckbereichen der Öffnungen 135 zusammen und sind dort miteinander verbunden oder einstückig ausgebildet. Von diesen Eckbereichen ragen die elastischen Haltezungen 140 in einer Anordnung mit dreizähliger Punktsymmetrie in die benachbarten Öffnungen 135 ab. Dies führt zu einer symmetrischen Kraftableitung beim Halten der Behälter über die Haltezungen 140. Die Haltezungen 140 bewirken so eine vorteilhafte Dreipunkt-Lagerung der Behälter in den Öffnungen, sodass die Behälter automatisch zentriert bezüglich einer Mittellinie 132 (vgl. Fig. 13d) einer jeweiligen Öffnung 135 gehalten werden.

Wie man der Fig. 13b entnehmen kann, ragen die Haltezungen 140 von den Seitenwänden 138 des Trägers 134 in Eckbereichen der Öffnungen 135 ab, also dort, wo die miteinander verbundenen oder einstückig ausgebildeten Seitenwände 138 Abschnitte mit vergleichsweise hoher Stabilität ausbilden. Zweckmäßig können in diesen Bereichen auch die vorgenannten Zapfen 143 angeformt bzw. ausgebildet sein.

Im Falle einer alternativen Ausführungsform, bei der die Seitenwände einer jeweiligen Öffnung oder Aufnahme jeweils kreisförmig und umlaufend ausgebildet sind, sind die Seitenwände ebenfalls bevorzugt miteinander verbunden oder einstückig ausgebildet. Die Haltezungen ragen dabei von den gleichen Bereichen ab wie bei der in der Fig. 13b dargestellten Anordnung. In diesen Bereichen können die Zwischenräume zwischen den kreisrund ausgebildeten Seitenwänden auch ausgefüllt sein.

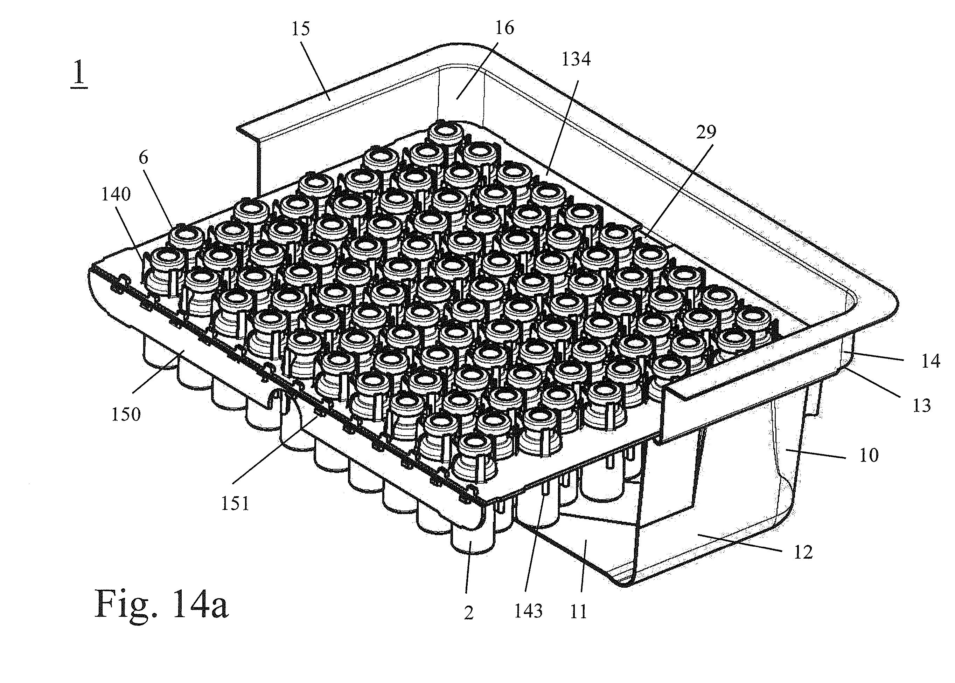

Die Fig. 13c zeigt einen Teilschnitt der Haltestruktur entlang A-A von Fig. 13b. Erkennbar ist, dass der Träger 134 auf der Unterseite von einem umlaufenden Rand 133 begrenzt ist, auf welchem der Träger 134 auf einer umlaufenden Stufe 13 (vgl. Fig. 14a) eines Transport- oder Verpackungsbehälter 1 abgestützt werden kann.

Die Fig. 13d zeigt einen stark vergrößerten Teilschnitt in dem Einsatz, der in der Fig. 13c dargestellt ist. Erkennbar ist, dass die Behälter ohne weiteres von unten her in die Öffnungen 135 des Trägers 134 eingeführt werden können. Beim Einführen der Behälter in die Öffnungen 135 kommt es zu einem elastischen Verbiegen der elastischen Haltezungen 140.

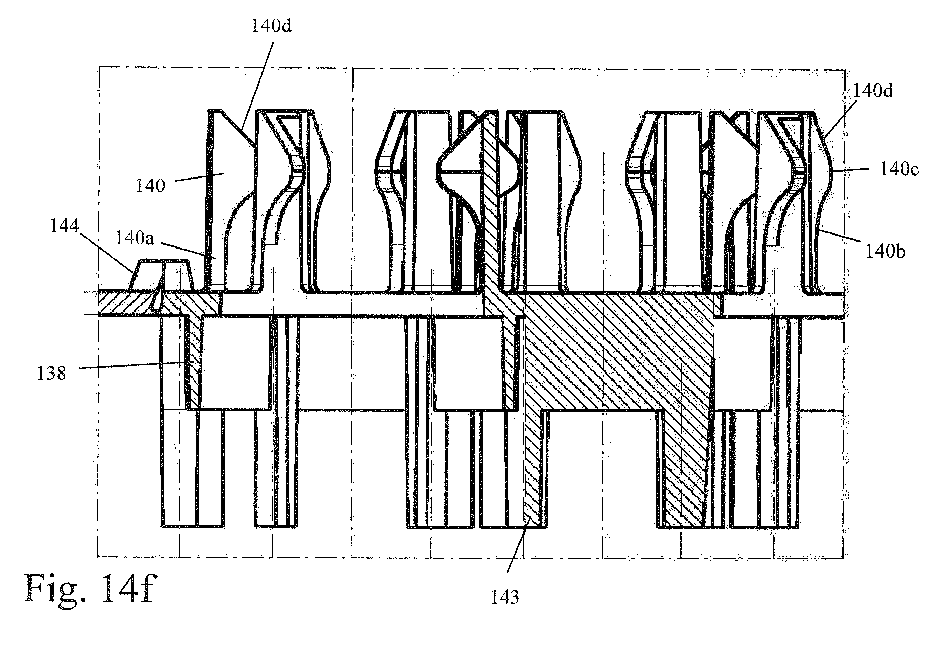

Je nach konkreter Ausgestaltung der zu haltenden Behälter können diese grundsätzlich auch von oben her in die Öffnungen 135 des Trägers 134 eingeführt werden, um an dem Träger 134 gehalten zu werden. Dies hat den Vorteil, dass das Risiko, dass Flüssigkeit oder anderer Behälterinhalt aus dem Behälterinnenraum der noch unverschlossenen Behälter unkontrolliert beim Einführen in die Öffnungen und beim Wegschwenken der Haltezungen 140 auf die Haltestruktur, insbesondere die Trägerplatte 134, gelangen können, noch weiter verringert werden kann. Zu diesem Zweck können auf der Oberseite der elastischen Haltezungen 140 Einführschrägen vorgesehen sein, wie diese nachfolgend näher anhand der Fig. 14f für eine alternative Ausführungsform beschrieben werden.

Durch Stärke, Material und Auslegung der elastischen Haltezungen 140 kann die zum Einführen und Entnehmen eines Behälters notwendige Kraft einfach vorgegeben werden.

Die Behälter sind zumindest mit radialem Spiel und bevorzugt sowohl mit radialem als auch mit axialem Spiel lose auf den Haltezungen abgestützt. Auf diese Weise können auch große Toleranzen von Behältern und unterschiedliche Außendurchmesser im Bereich des Halsabschnitts 5 einfach ausgeglichen werden. Denn zur Halterung der Behälter genügt es, wenn der Rollrand 6 noch auf den Oberseiten der Haltezungen 140 aufliegt. Grundsätzlich können dadurch auch Behälter unterschiedlichen Typs, beispielsweise mit unterschiedlichen Durchmessern im Bereich des Halsabschnitts 5, von derselben Haltestruktur gehalten werden.

Die Fig. 13e verdeutlicht dies in dem gleichen stark vergrößerten Teilschnitt wie nach der Fig. 13d und stellt die Halterung eines Behälters in einer Öffnung 135 des Träges 134 dar. Gemäß der Fig. 13e liegt die Unterseite des verbreiterten Rands 6 auf dem vorderen Ende der elastischen Haltezungen 140 im Übergangsbereich zwischen dem verengten Halsabschnitt 5 und dem Rand 6 lose auf, um die Lage des Behälters zu fixieren. Wie man in der Fig. 13e erkennen kann, besteht zwischen den Haltezungen 140 (vgl. linker Bildteil) und dem verengten Halsabschnitt 5 ein Luftspalt, der ein radiales Spiel ermöglicht. Aufgrund dieser Halterung mit radialem Spiel besteht dabei, je nach konkreter Ausbildung des Behälters, noch die Möglichkeit, den von den Haltezungen 140 gehaltenen Behälter axial zu verschieben, d.h. in Längsrichtung der Behälter, beispielsweise solange, bis die Böden 3 von allen von dem Träger 134 gehaltenen Behältern unter dem gleichen Abstand zum Träger 134 gehalten werden, um gemeinsam eine Ebene aufzuspannen.

Gemäß der Fig. 13e ist der Behälter soweit in die Öffnung 135 eingeschoben, dass der verbreiterte Rand 6 auf den vorderen Enden der Haltezungen genau an dem Übergangsbereich zwischen dem verengten Halsabschnitt 5 und dem verbreiterten oberen Rand 6 abgestützt ist. Dies lässt sich beispielsweise durch Einschieben der Behälter von unten her in die Öffnungen 135 des Trägers 134 und anschließendes Herabdrücken der Behälter bewerkstelligen, und zwar solange, bis die vorderen Enden der Haltezungen genau an dem Übergangsbereich zwischen dem verengten Halsabschnitt 5 und dem verbreiterten oberen Rand 6 anliegen. In der Haltestellung gemäß der Fig. 13e ist jedenfalls bei der großen Mehrzahl der fixierten Behälter ein gewisser radialer Abstand zwischen dem stufenartigen Übergangsbereich zwischen dem oberen Rand 6 und dem verengten Halsabschnitt 5 und vorderen Ende der Haltezungen 140 vorgesehen. Auf diese Weise können in einem gewissen Umfang Fertigungstoleranzen der Behälter in axialer Richtung und auch Fertigungstoleranzen in radialer Richtung kompensiert werden und somit auch Behälter mit unterschiedlichen Durchmessern im Bereich des verengten Halsabschnitts 5 von ein- und demselben Träger 134 gehalten werden. Damit lassen sich auch etwaige Verspannungen im Kunststoff des Trägers 134 aufgrund der Aufnahme von Behältern mit einem zu großen Außendurchmesser gering halten.

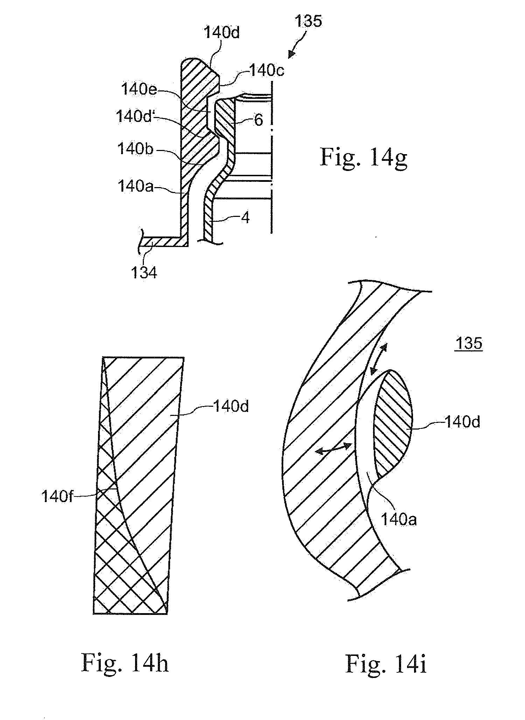

Gemäß einer alternativen Ausführungsform, wie nachfolgend anhand der Fig. 14g beschrieben, können die Behälter auch formschlüssig an dem Träger 134 gehalten werden.

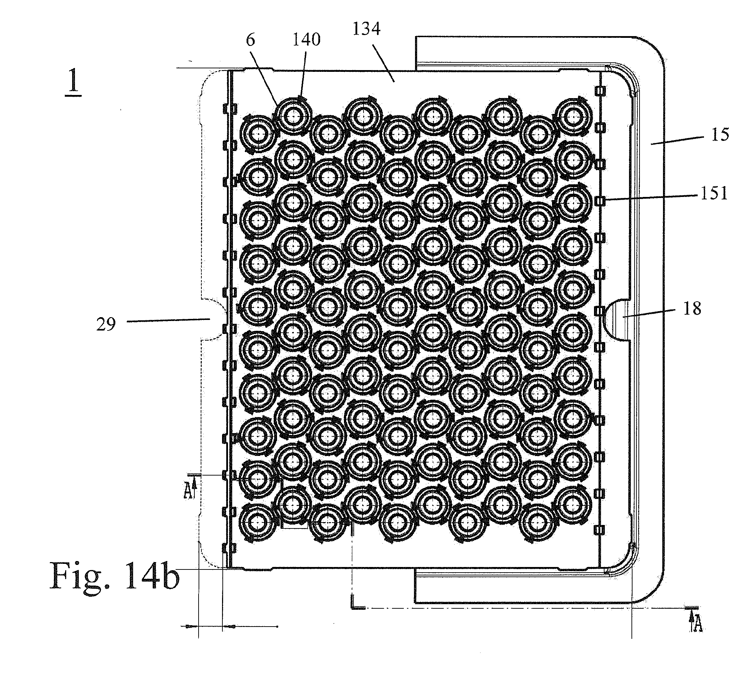

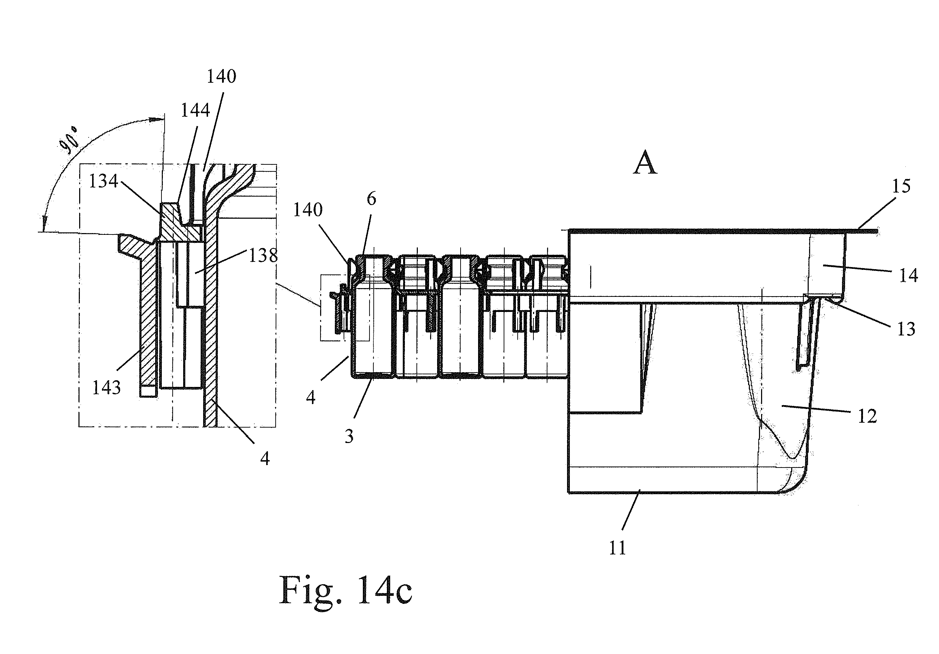

Zum Transport und zur Verpackung der vorstehend beschriebenen Haltestruktur mit den darin aufgenommenen Behältern dient ein Transport- und Verpackungsbehälter 10 (im Stand der Technik auch als "Tub" bezeichnet), wie dieser grundsätzlich anhand der Fig. 14c vorstehend beschrieben wurde und im Zusammenhang mit einer Darstellung einer weiteren Ausführungsform einer Haltestruktur gemäß der vorliegenden Erfindung in den Figuren 14a bis 14c gezeigt ist. Dabei zeigt die Fig. 14c in zwei vergrößerten Teilschnitten entlang A-A gemäß der Fig. 14b die Halterung von Behältern in der Haltestruktur gemäß der weiteren Ausführungform sowie Details davon. Erkennbar ist insbesondere, dass auf der Oberseite des Trägers abgeschrägte Anschlagnasen 144 vorgesehen sind, welche das Zurückschwenken der elastischen Haltezungen 140 beim Einführen der Behälter begrenzen.

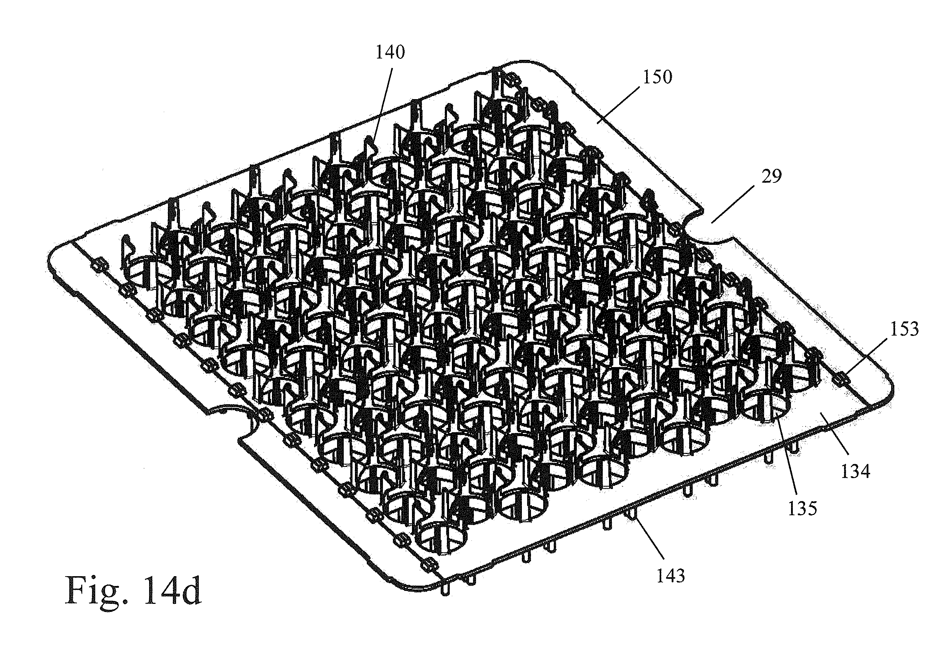

Die Fig. 14d zeigt in einer perspektivischen Draufsicht die Haltestruktur gemäß der Fig. 14a ohne Behälter. Erkennbar sind die elastischen Haltezungen 140 fähnchenartig und mit einer radial einwärts vorstehenden Haltenase ausgebildet, wie in dem stark vergrößerten Teilschnitt durch diese Haltestruktur gemäß der Fig. 14f besser dargestellt. Gemäß der Fig. 14f sind die elastischen Haltezungen 140 über eine senkrecht von der Oberseite des Trägers 134 abragende, elastische Basis 140a mit dem Träger 134 verbunden. Die Basis 140a geht in einen radial einwärts gekrümmten Abschnitt 140b über, der schließlich in die Haltenase 140c übergeht, auf welcher der verbreiterte Rand 6 (vgl. Fig. 13e) der Behälter aufliegt, wie vorstehend anhand der Fig. 13e für die erste Ausführungsform beschrieben. Die Haltenase 140c ragt dabei in die Öffnung des Trägers 134 hinein. Die Haltenase 140c geht in eine sich schräg aufwärts erstreckende Einführschräge 140d über, die mit dem oberen Ende der Haltezunge 140 verbindet. Aufgrund der Einführschräge 140d auf der Oberseite der Haltezunge 140 sowie des nach unten geöffneten, gekrümmten Abschnitts 140b der Haltezunge 140 können die Behälter wahlweise von oben oder von unten her in die Öffnungen des Trägers 134 eingeführt und aus diesen wieder abgezogen werden.

Beim Einführen der Behälter von oben her in die Öffnungen geraten zunächst die Böden bzw. unteren Enden der Behälter in Anlage zu den Einführschrägen 140d der Haltezungen 140. Beim weiteren Einführen der Behälter gleitet das untere Ende bzw. der Boden der Behälter entlang den Einführschrägen 140d abwärts und spreizt dabei die Haltezungen 140 zunehmend elastisch auseinander oder klappt bzw. schwenkt diese zurück. Beim weiteren Einführen der Behälter gerät schließlich die zylindrische Seitenwand der Behälter (vgl. Fig. 13e) in Anlage zu den Haltenasen 140c und gleitet an diesen entlang, solange bis schließlich die Unterseite des verbreiterten Rands der Behälter lose auf den Haltenasen 140c der Haltezungen 140 aufliegt. Die Behälter können dann entweder nach oben hin mit umgekehrtem Bewegungsablauf der Haltezungen 140 und ohne elastisches Verbiegen der Haltezungen 140 oder nach unten hin mit elastischem Verbiegen der Haltezungen 140 aus den Öffnungen des Trägers 134 entnommen werden.

Beim Einführen der Behälter von unten her in die Öffnungen gerät das obere Ende der Behälter zunächst in Anlage zu dem gekrümmten Abschnitt 140b der Haltezungen. Beim weiteren Einführen der Behälter gleitet das obere Ende der Behälter entlang den gekrümmten Abschnitten 140b aufwärts und spreizt dabei die Haltezungen 140 zunehmend elastisch auseinander oder klappt bzw. schwenkt diese zurück, bis schließlich die Haltenasen 140c erreicht sind. Beim weiteren Hochschieben der Behälter gleitet die Unterseite des verbreiterten Rands der Behälter über die Haltenasen 140c der Haltezungen 140 und liegt schließlich lose auf den Haltenasen 140c der Haltezungen 140 auf. Die Behälter können dann entweder nach unten hin mit umgekehrtem Bewegungsablauf der Haltezungen 140 und mit elastischem Verbiegen der Haltezungen 140 oder nach oben hin ohne elastisches Verbiegen der Haltezungen 140 aus den Öffnungen des Trägers 134 entnommen werden.

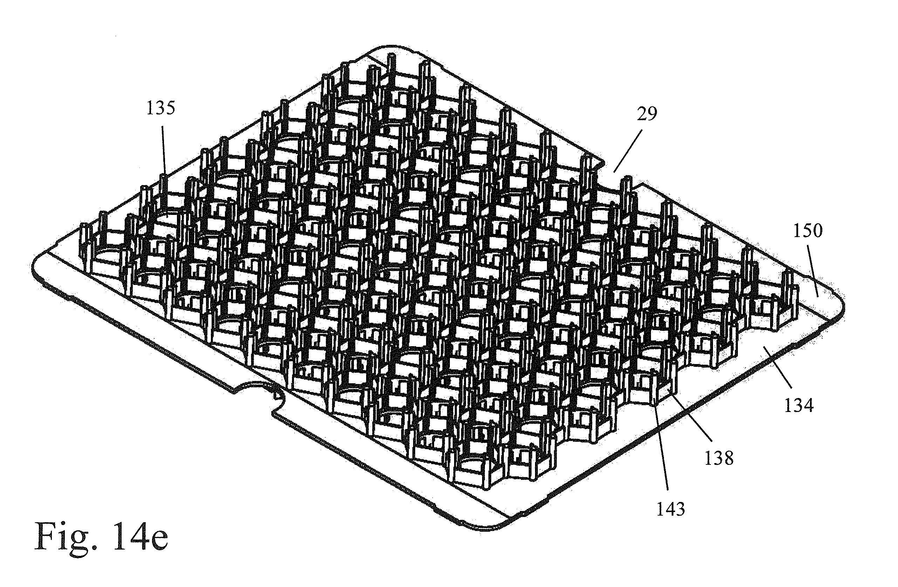

Die Fig. 14e zeigt in einer perspektivischen Unteransicht die Haltestruktur gemäß der Fig. 14a ohne Behälter. Erkennbar ist die wabenförmige, hexagonale Anordnung der umlaufenden Seitenwände 138, in deren Eckbereichen Zapfen 143 senkrecht von der Unterseite des Trägers 134 abragen. Diese Zapfen 143 dienen als Abstandshalter beim Ablegen des Trägers 134 auf einer Ablagefläche, beispielsweise dem Boden 11 eines Transport- und Verpackungsbehälters (vgl. Fig. 14a), vermeiden aber gleichzeitig auch den Kontakt der Behälter untereinander.

Die Fig. 14g zeigt in einem stark vergrößerten Teilschnitt die Halterung eines Behälters in einer Haltestruktur gemäß einer weiteren Ausführungsform der vorliegenden Erfindung. Abweichend zur vorgenannten Ausführungsform werden die Behälter hier an ihrem verbreiterten oberen Randabschnitt 6 (Rollrand) formschlüssig umgriffen, wobei ein ausreichendes radiales Spiel, wie vorstehend beschrieben, gewährleistet ist, wie durch den Luftspalt in Radialrichtung in der Fig. 14g angedeutet. Alternativ kann zusätzlich zu diesem radialen Spiel auch ein ausreichendes axiales Spiel gewährleistet sein, wie durch den Luftspalt in axialer Richtung in der Fig. 14g angedeutet. Zu diesem Zweck ist am vorderen Ende der Haltenase 140c (vgl. Fig. 14f) eine C-förmige Aussparung 140e vorgesehen, die über Schrägen 140d' in die Haltenase 140c übergeht. In der Haltestellung gemäß der Fig. 14g liegt der verbreiterte Randabschnitt 6 lose und mit radialem Spiel auf der unteren Schräge 140d' der Aussparung 140e auf. Wie in der Fig. 14g dargestellt, kann zwischen dem oberen Ende des verbreiterten Randabschnitts 6 und der oberen Schräge 140d' der Aussparung ein ausreichendes axiales Spiel bereitgestellt sein. Insgesamt wird somit der verbreiterte Randabschnitt 6 von der Haltezunge 140 klammerartig und formschlüssig umgriffen. Die Einführschräge 140d', der gekrümmte Abschnitt 140b sowie die Schrägen 140d' der Aussparung ermöglichen dabei ein Einführen und Abziehen der Behälter ohne größeren Kraftaufwand in die Öffnungen bzw. aus diesen heraus unter elastischem Wegbiegen der Haltezungen 140.

Die Fig. 14h stellt eine stark vergrößerte Draufsicht auf eine Einführschräge einer Haltezunge gemäß einer Variante zu der Haltestruktur gemäß der Fig. 14a dar. Gemäß der Fig. 14h ist die Einführschräge 140d mittels eines darauf ausgebildeten bogenförmigen Grats 140f insgesamt verdrillt ausgebildet. Diese gewundene Einführschräge 140d ist auf sämtlichen Haltezungen der Öffnungen oder Aufnahmen gleich ausgebildet. Insgesamt sind die Einführschrägen, in Draufsicht betrachtet, um einen Winkel von kleiner 90° gekrümmt ausgebildet. Im Zusammenwirken mit dem Behälter bewirkt dies beim Einführen der Behälter in die Öffnungen, dass die Haltezungen nicht nur radial auswärts weggeschwenkt oder zurück geklappt werden, sondern gleichzeitig mit einer Bewegungskomponente in Umfangsrichtung in Entsprechung zu der Geometrie der Einführschrägen 140d weggedreht werden, und zwar um einen Winkel von kleiner 90°. Je nach der Geometrie der Anordnung der Haltezungen auf dem Träger kann so eine Kollision von Haltezungen von unmittelbar benachbarten Öffnungen oder Aufnahmen beim Zurückschwenken oder Zurückklappen vermieden werden. Auf diese Weise kann die Packungsdichte der Behälter an der Haltestruktur noch weiter erhöht werden.

Die Fig. 14i zeigt in einer Draufsicht eine weitere Variante von Haltezungen für eine Haltestruktur gemäß der Fig. 14a, bei der die Basis 140a, in Axialrichtung betrachtet, verdrillt ausgebildet ist, was im Zusammenwirken der Einführschräge 140d mit dem Behälter beim Einführen des Behälters von oben her in die Öffnung oder Aufnahme sowohl eine radiale Komponente als auch eine Komponente in Umfangsrichtung beim elastischen Wegschwenken der Haltezungen ergibt, wie durch die beiden Doppelpfeile schematisch angedeutet.

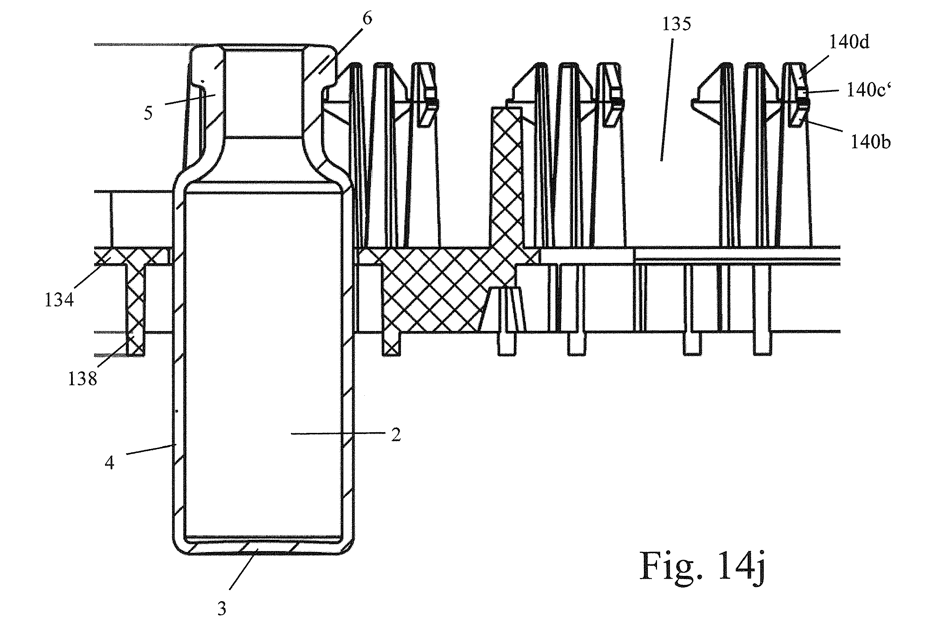

Die Fig. 14j zeigt in einer schematischen Schnittansicht eine weitere Variante von Haltezungen für eine Haltestruktur gemäß der Fig. 14a, bei der unterhalb der oberen Einführschräge 140d eine sich im Wesentlichen senkrecht erstreckende Aussparung 140c' ausgebildet ist, welche in eine Stufe übergeht, auf der die Unterseite des oberen Rands des aufzunehmenden Behälters unmittelbar aufliegt. Der Stufe schließt sich die untere Einführschräge 140b in der vorstehend beschriebenen Weise an.

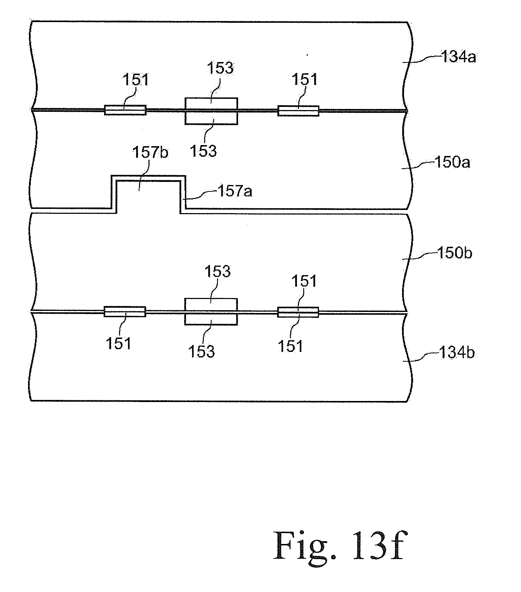

Die Fig. 13f zeigt in einem stark vergrößerten Teilschnitt und in Draufsicht eine weitere Variante der Haltestruktur gemäß der Fig. 13b, wobei Ränder 150a, 150b des plattenförmigen Trägers 134a, 134b wegeklappt werden können, um die Grundfläche des jeweiligen Trägers weiter zu reduzieren, beispielsweise dann, wenn dieser mit den Behältern an eine beengte Weiterverarbeitungsstation übergeben werden soll, beispielsweise an einen Gefriertrockenschrank mit begrenzter Grundfläche. Zu diesem Zweck sind die Ränder 150a, 150b über Scharniere 151 mit dem jeweiligen Träger verbunden. Insbesondere können die Scharniere 151 als Filmscharniere oder Schnapp- bzw. Federscharniere aus einem Kunststoff einstückig mit dem Träger 134 ausgebildet sein.

Gemäß der Fig. 13f sind an den abnehmbaren oder wegschwenkbaren Elementen 150a, 150b Aussparungen 157a und/oder Vorsprünge 157b ausgebildet. Die Aussparungen 157a und/oder Vorsprünge 157b der abnehmbaren oder wegschwenkbaren Elemente 150 eines Trägers sind korrespondierend zu den Aussparungen 157a und/oder Vorsprüngen 157b der abnehmbaren oder wegschwenkbaren Elemente 150 eines unmittelbar benachbarten flächigen Trägers ausgebildet, sodass ein Formschluss zwischen diesen Aussparungen 157a und/oder Vorsprüngen 157b ausgebildet werden kann, um die gegenseitige Lage der Träger festzulegen und zu stabilisieren.

Auf der Oberseite der Träger 134a, 134b und der Ränder 150a, 150b sind an einander entsprechenden Positionen blockförmige Anschläge 153 vorgesehen, die im gegenseitigen Anschlag eine koplanare Ausrichtung der Ränder 150a, 150b und des Trägers 134 festlegen und ein Hochklappen der Ränder 150a, 150b verhindern. Die Träger können deshalb auch nur an den Rändern in einem Transport- und Verpackungsbehälter (vgl. Fig. 14a) abgelegt werden.

Gemäß einer weiteren Ausführungsform (nicht dargestellt) können die Ränder 150 auch von dem Träger 134 abgenommen werden. Die Ränder 150 können selbstverständlich entlang von allen vier Längsseiten des Trägers 134 vorgesehen sein.

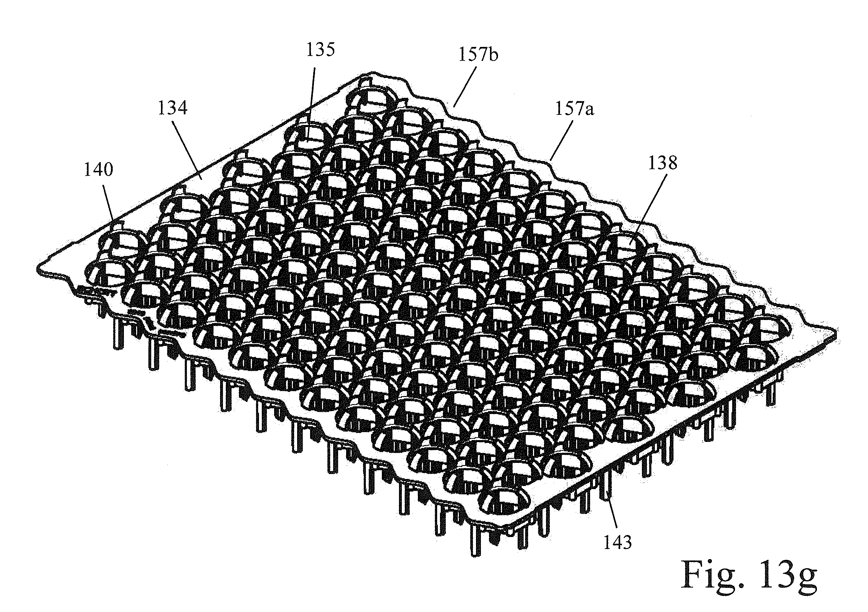

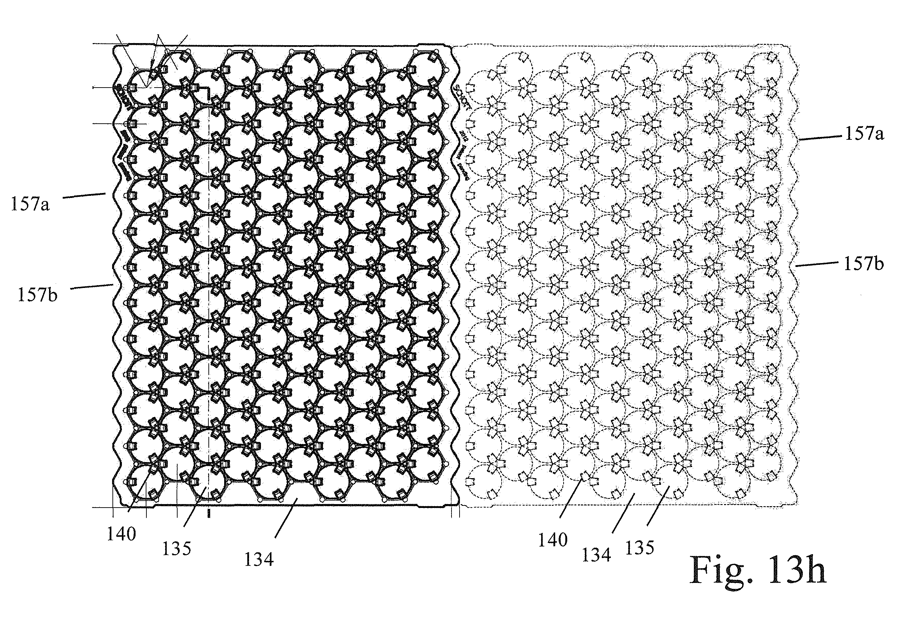

Die Fig. 13g zeigt eine weitere Variante der vorgenannten Haltestruktur gemäß der Fig. 13f, wobei die vorgenannten Vorsprünge 157b und Aussparungen 157a unmittelbar am Rand des flächigen Trägers 134 ausgebildet sind. Die Fig. 13h zeigt das Zusammenwirken der Vorsprünge 157b und Aussparungen 157b von zwei benachbarten Trägern 134, sodass diese ineinander greifen und eine Relativverschiebung zwischen den beiden Trägern 134 verhindert wird. In dieser Stellung können benachbarte Träger 134 gemeinsam transportiert und verschoben werden, beispielsweise in einem Sammlungsbereich vor einem Gefriertrockenschrank, wie beispielhaft in der Fig. 11 abgebildet.

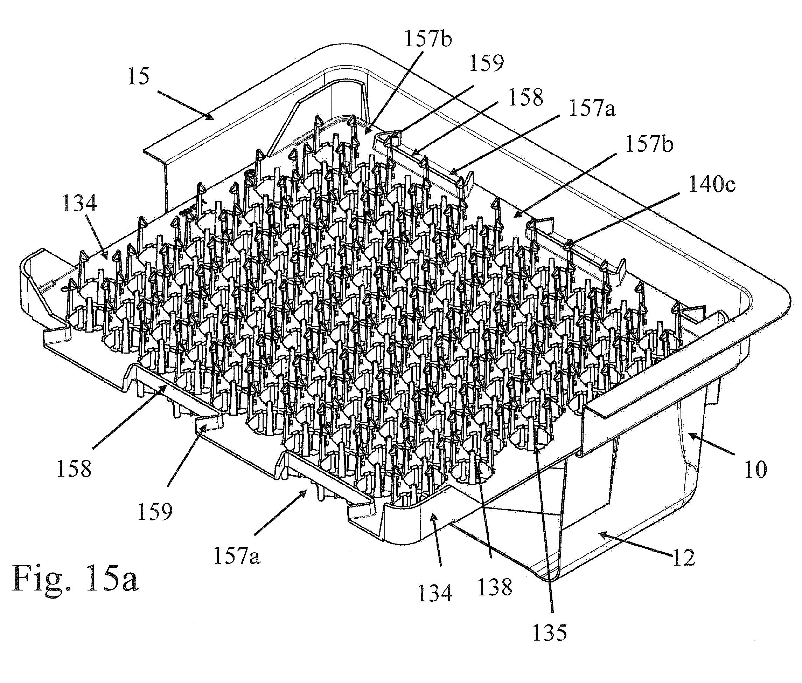

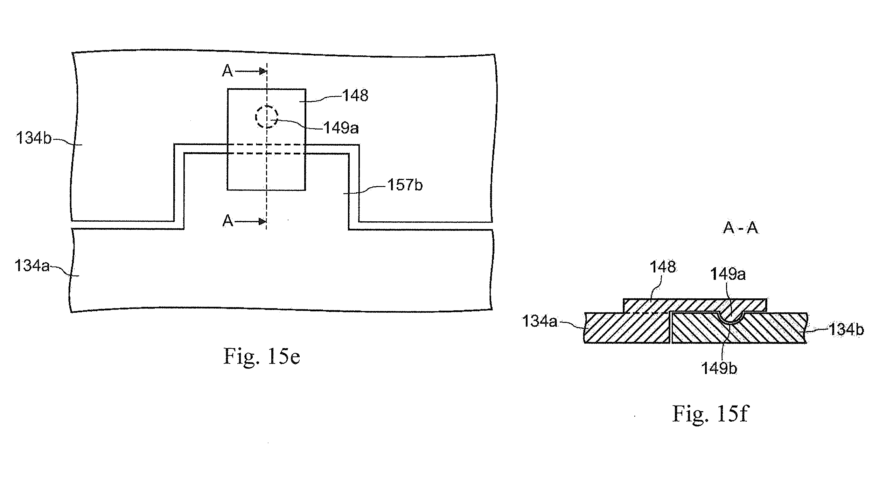

Die Fig. 15a zeigt eine Haltestruktur gemäß einer weiteren, auch unabhängig beanspruchbaren Ausführungsform der vorliegenden Erfindung in einer perspektivischen Draufsicht. Gemäß der Fig. 15a sind entlang der beiden Längsseiten der Halteplatte 134 alternierend und unter regelmäßigen Abständen zueinander eine Mehrzahl von Vorsprüngen 157b und Aussparungen 157a ausgebildet. Diese weisen, jeweils in Draufsicht betrachtet, eine insgesamt dreieckförmige oder polyedrische Grundfläche auf und sind korrespondierend zueinander ausgebildet, sodass diese unmittelbar miteinander verhakt werden können.

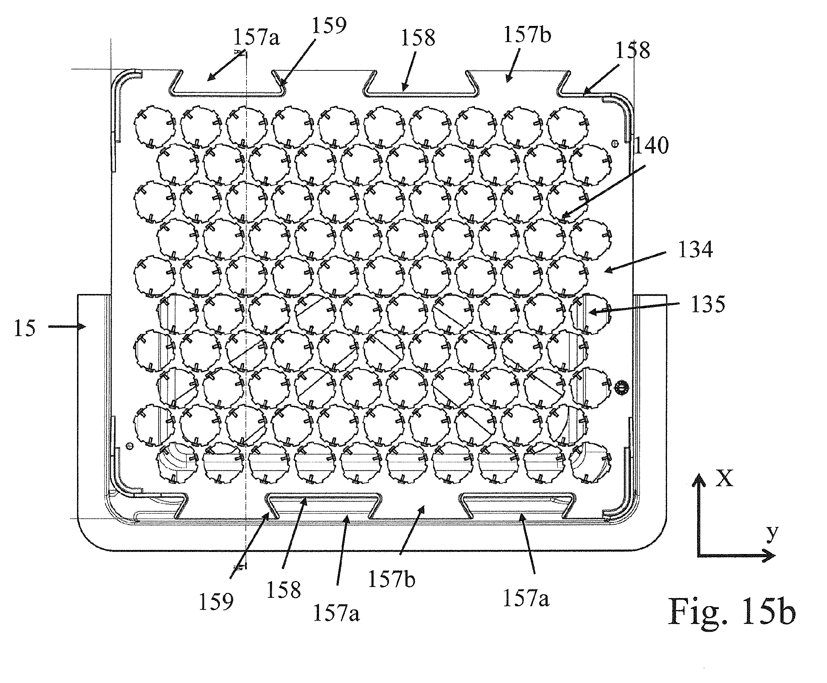

Wie man der Draufsicht gemäß der Fig. 15b entnehmen kann, können zwei Haltestrukturen so miteinander verhakt werden, dass diese in der Querrichtung (x) fluchtend angeordnet sind. Zu diesem Zweck ist im rechten unteren Eckbereich der Halteplatte 134 die Aussparung 157a nur halb ausgebildet. Im gegenüber liegenden rechten oberen Eckbereich der Halteplatte 134 ist hingegen der korrespondierende Vorsprung 157b ebenfalls nur halb ausgebildet und geht über in eine abgerundete Ecke der Halteplatte 134.

Durch die vorgenannte Auslegung der Vorsprünge 157b und Aussparungen 157a können zwei Haltestrukturen jedoch grundsätzlich auch so miteinander verhakt werden, dass diese in der Querrichtung (x) versetzt zueinander, also nicht-fluchtend, angeordnet sind.

Zum Verhaken zweier Haltestrukturen kann eine der Haltestrukturen mittels einer Hubeinrichtung in einer Richtung senkrecht zur Ebene der Halteplatte 134 angehoben werden. Anschließend werden die beiden Haltestrukturen aufeinander zubewegt, bis schließlich, in Draufsicht betrachtet, die Vorsprünge 157b und Aussparungen 157a der benachbarten Haltestrukturen miteinander überlappen. Durch anschließendes Absenken der Halteplatte 134 senkrecht zur Ebene der Halteplatte 134 greifen schließlich die Vorsprünge 157b und Aussparungen 157a formschlüssig ineinander ein. Diese Vorgehensweise kann manuell aber auch voll- oder halbautomatisch erfolgen. Dabei können die Halteplatten 134 bereits mit Fläschchen bestückt sein. Grundsätzlich kann die Bestückung der Halteplatten 134 mit Fläschchen jedoch auch erst dann erfolgen, wenn die Halteplatten 134 miteinander verbunden sind.

Durch die vorgenannte Auslegung der Vorsprünge 157b und Aussparungen 157a wird insgesamt eine einhakende Wirkung in der Art einer Schwalbenschwanzverbindung realisiert. Wie dem Fachmann beim Studium der vorstehenden Beschreibung ohne weiteres ersichtlich sein wird, können grundsätzlich auch beliebige andere form- oder kraftschlüssige Verbindungstechniken zur vorübergehenden, lösbaren Verbindung zweier Haltestrukturen eingesetzt werden.

Wie man der perspektivischen Draufsicht gemäß der Fig. 15a entnehmen kann, sind entlang den Rändern der Vorsprünge 157b und Aussparungen 157a zumindest abschnittsweise Seitenwände 158, 159 ausgebildet, die rechtwinklig von der Oberfläche der Halteplatte 134 abragen. Diese Seitenwände 158, 159 folgen der Kontur der zugeordneten Aussparung 157a bzw. des zugeordneten Vorsprungs 157b und wirken als Anschlag- und Führungsfläche, die ein Übereinandergleiten bzw. Aufschieben der Halteplatten 134 verhindern. Genauer gesagt ist gemäß der Fig. 15b entlang der Vorderseite der Vorsprünge 157b am oberen Rand der Halteplatte 134 eine Seitenwand 158 ausgebildet, der sich im Bereich der benachbarten Aussparungen 157a eine Seitenwand 159 anschließt, die sich jedoch nicht über die gesamte Tiefe der Aussparungen (in x-Richtung) erstreckt. Am gegenüber liegenden unteren Rand der Halteplatte 134 sind die Seitenwände 158 dagegen entlang der Basis der Aussparungen 157a ausgebildet, während sich die abgewinkelten Seitenwände 159a entlang den abgewinkelten Seiten der Aussparungen 157a erstrecken, nicht jedoch über deren gesamte Tiefe (in x-Richtung).

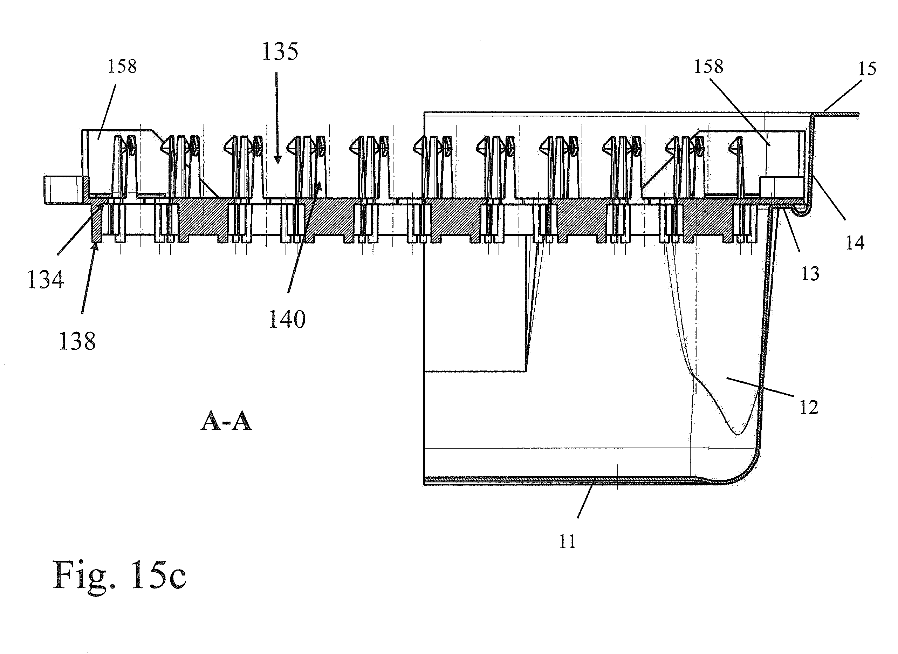

Wie in der stark vergrößerten Teildraufsicht gemäß der Fig. 15c dargestellt, liegen im verhakten Zustand die Seitenwände 158a der unteren Halteplatte 134a unmittelbar an den Seitenwänden 158b der obere Halteplatte 134b an. Ferner liegen auch die abgewinkelten Seitenwände 159b der oberen Halteplatte 134b unmittelbar an den abgewinkelten Seitenwände 159a der unteren Halteplatte 134a an.

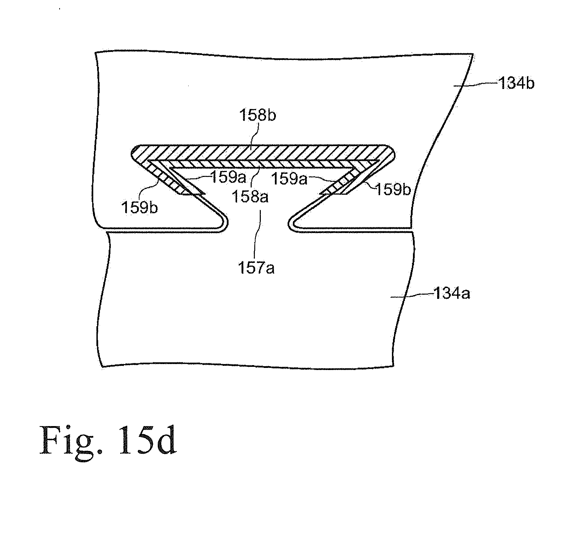

Die Fig. 15d zeigt als weiteres Beispiel für eine formschlüssige Verbindung in einer stark vergrößerten Teildraufsicht die Verbindung zweier Halteplatten 134a, 134b nach einer weiteren Ausführungsform. Gemäß der Fig. 15d ragt von den hier rechteckförmig ausgebildeten Vorsprüngen 157b der unteren Halteplatte 134a eine elastische Zunge 148 in Richtung der zugeordneten Aussparung der oberen Halteplatte 134b rechtwinklig ab. Wie sich dem schematischen Teilschnitt gemäß der Fig. 15e entlang der Linie A-A gemäß der Fig. 15d entnehmen lässt, steht die elastische Zunge von der von den Halteplatten 134a, 134b aufgespannten Ebene vor, erstreckt sich jedoch parallel zu diesen. Am vorderen Ende der elastischen Zunge 148 ist ein kugelförmiger Vorsprung 149a ausgebildet, der in eine korrespondierend ausgebildete Aufnahme 149b auf der Oberseite der oberen Halteplatte 134b eingreift. Die Halteplatten 134a, 134b können zur Verbindung aufeinander zu geschoben werden, bis das vordere Ende der elastischen Zunge 148 mit dem Vorsprung 149a schließlich in Anlage mit der Oberseite der oberen Halteplatte 134b gelangt. Bei weiterer Annäherung der beiden Halteplatten 134a, 134b wird schließlich die elastische Zunge 148 nach oben gebogen, sodass der Vorsprung 149a entlang der Oberfläche der oberen Halteplatte 134b gleitet, bis dieser schließlich in den Bereich der Aufnahme 149b gelangt und aufgrund der Rückstellkraft der elastischen Zunge 148 in diese gedrückt wird. Die Elastizität der Zungen 148 und die Ausgestaltung der Formschlussgebilde 149a, 149b legen dabei in einfacher Weise die Stärke der lösbaren Verbindung zwischen den beiden Halteplatten 134a, 134b fest. Um ein Aufgleiten der beiden Halteplatten 134a, 134b zu verhindern, können auch nach dieser Ausführungsform Anschlag- und Führungsflächen vorgesehen sein, insbesondere in Gestalt von Seitenwänden, die rechtwinklig von der Oberseite der Halteplatten 134a, 134b abragen, wie vorstehend anhand der Fig. 15a beschrieben. Bei der Ausführungsform nach der Fig. 15d wären solche Seitenwände insbesondere seitlich neben den elastischen Zungen 148 vorzusehen.

Wie dem Fachmann beim Studium der vorstehenden Beschreibung ohne weiteres ersichtlich sein wird, ist der vorgenannte Gesichtspunkt der form- oder kraftschlüssigen Verbindung von unmittelbar benachbarten Haltestrukturen grundsätzlich unabhängig von der konkreten Ausführung der Halterung der Fläschchen an solchen Haltestrukturen, sodass dieser Gesichtspunkt grundsätzlich auch als unabhängiger Gesichtspunkt der vorliegenden Erfindung unabhängig von der konkreten Realisierung der Halterung der Fläschchen an solchen Haltestrukturen beansprucht werden kann.

Das Verfahren nach der Erfindung beruht maßgeblich darauf, dass eine Mehrzahl von Behältern gemeinsam an einem Träger gehalten werden und, während diese an dem Träger gehalten oder zumindest geführt sind, behandelt oder weiter verarbeitet werden können. Wie dem Fachmann bei Studium der vorstehenden Beschreibung ohne Weiteres ersichtlich sein wird, eignet sich dieser Lösungsansatz grundsätzlich für beliebige Prozessschritte zur Behandlung oder Verarbeitung von Behältern zur Aufbewahrung von Substanzen für kosmetische, medizinische oder pharmazeutische Anwendungen.

Die von den reibschlüssig oder formschlüssig wirkenden Haltemitteln eines Trägers jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Gemäß weiteren Ausführungsformen kann die Haltekraft durch geeignete Auslegung der Haltemittel auch so bemessen sein, dass diese größer ist als übliche Kräfte bei der Handhabung, Verarbeitung oder Behandlung der Behälter in einer Prozessanlage. Dadurch wird stets eine zuverlässige Halterung der Behälter gewährleistet. Gleichwohl können die Behälter gemäß bevorzugten weiteren Ausführungsformen der Erfindung gegen diese Haltekraft in den Öffnungen oder Aufnahmen verstellt werden, insbesondere axial vorgeschoben oder gedreht werden. Die hierfür erforderliche Kraft muss nur größer sein als die von den Haltemitteln ausgeübte Kraft.

Zum Einführen, Herausnehmen oder Verschieben der Behälter an einem Träger muss diese Haltekraft zunächst überwunden werden. Dies hat den Vorteil, dass die Behälter bei Einwirkung einer geringen Kraft, etwa durch Erschütterungen der Prozessanlage oder von Fördermitteln derselben, weiterhin zuverlässig an dem Träger gehalten werden und nicht etwa versehentlich umfallen. Dies mindert die Gefahr von unbeabsichtigten Verunreinigungen in Prozessanlagen erheblich. So kleben in die Öffnung von Behältern eingebrachte Stopfen häufig im Laufe einer Behandlung an und können danach nicht rüttelfrei verstellt werden, beispielsweise um einen Behälter zu verschließen oder diesen zu öffnen. Herkömmlich hat dies häufig zu einem Umfallen von Behältern und zu einem unerwünschten Auslaufen von Substanzen in Prozessanlagen geführt. Da die Behälter mit einer vorbestimmten Minimum-Haltekraft an den Trägern gehalten sind, ist dieses Risiko bei einem Verfahren nach der Erfindung erheblich minimiert.

Herkömmlich wurden deshalb Stopfen oder vergleichbare elastische Verschlussteile mit einer Gleitbeschichtung versehen, was zu unerwünschten Verunreinigungen führte. Grundsätzlich kann bei einem Verfahren nach der Erfindung ohne solche Gleitbeschichtungen gearbeitet werden, sodass Wirkstoffe mit einem Verfahren nach der Erfindung in noch reinerer Form verarbeitet und behandelt werden können.

Selbstverständlich kann die Haltestruktur (der Träger) im Sinne der vorliegenden Erfindung auch aus einem thermoplastischen, duroplastischen oder elastomeren Kunststoff ausgebildet sein, zumindest Abschnitte der Haltestruktur bzw. des Trägers mit einer reibreduzierenden Beschichtung versehen werden, um das Einführen und die Entnahme der Behälter zu erleichtern.

Gemäß weiteren Ausführungsformen kann die Haltestruktur und/oder der Transportbehälter, oder Abschnitte davon, aus einem faserverstärkten Kunststoff oder einem Kunststoff ausgebildet sein, dem zur Erhöhung seiner Wärmeleitfähigkeit Keramiken oder Metalle beigegeben sind. Bekanntermaßen haben faserverstärkte Kunststoffe eine höhere Wärmeleitfähigkeit bis 0,9 W/(K m) bei Kohlenstofffasern. Werden den Kunststoffen Keramiken oder Metalle beigegeben, so wird die Wärmeleitfähigkeit weiter vergrößert. Es entstehen die sogenannten wärmeleitenden Kunststoffe. So wird eine Wärmeleitfähigkeit von 20 W/(K m) erreicht.

Wie dem Fachmann beim Studium der vorstehenden Beschreibung ohne Weiteres ersichtlich sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorstehenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Verfahren zur Behandlung oder Verarbeitung von Behältern (2), die zur Aufbewahrung von Substanzen für medizinische, pharmazeutische oder kosmetische Anwendungen dienen oder diese enthalten, wobei die Behälter an einem Ende offen ausgebildet sind und mittels einer Fördereinrichtung (221) zur Behandlung oder Verarbeitung automatisiert an Prozessstationen vorbeigeführt werden oder diese durchlaufen, bei welchem Verfahren
eine Mehrzahl von Behältern (2) von der Fördereinrichtung (221) gefördert wird, während diese von einem Träger (25; 134) gemeinsam in einer regelmäßigen zweidimensionalen Anordnung gehalten werden, wobei der Träger eine Mehrzahl von Öffnungen oder Aufnahmen (32; 39; 87; 120) aufweist, welche die regelmäßige Anordnung vorgeben, und wobei die Böden (3) der Behälter an deren geschlossenen Enden von einer ersten Seite des Trägers her frei zugänglich sind,
**dadurch gekennzeichnet, dass** eine Prozessstation ein Gefriertrockenschrank (220) mit zumindest einer Kühlfläche (223) ist, in welchem ein Gefriertrocknungsprozess ausgeführt wird, wobei die Böden (3) der Behälter während des Gefriertrocknungsprozesses in dem Gefriertrockenschrank in einem unmittelbaren Kontakt zu der jeweiligen Kühlfläche (223) stehen, während die Behälter von dem Träger gehalten werden oder in den Öffnungen oder Aufnahmen des Trägers aufgenommen sind.

2. Verfahren nach Anspruch 1, wobei die Träger (25; 134) in Transport- und Verpackungsbehältern (1) angeordnet sind und zum Ausführen des Gefriertrocknungsprozesses aus dem jeweiligen Transport- und Verpackungsbehälter entnommen und auf der jeweiligen Kühlfläche (223) abgestützt werden oder wobei die Träger (25; 134) während der Behandlung oder Verarbeitung in oder an zumindest einer der Prozessstationen in Transport- und Verpackungsbehältern (1) angeordnet sind.

3. Verfahren nach einem der vorhergehenden Ansprüche, wobei Randabschnitte (68) des Trägers abgenommen oder weggeschwenkt werden, um die Grundfläche des Trägers insgesamt zu verringern, wenn die Behälter in oder an der Prozessstation behandelt oder verarbeitet werden oder um den Gefriertrocknungsprozess auszuführen.

4. Verfahren nach einem der vorhergehenden Ansprüche, wobei jeweils unmittelbar benachbarte Träger so miteinander unmittelbar gekoppelt oder verbunden werden, dass diese relativ zueinander in einer Längsrichtung und/oder in Querrichtung der jeweiligen Träger vorübergehend unverschieblich sind.

5. Verfahren nach einem der vorherigen Ansprüche, wobei die Behälter (2) zur Behandlung oder Verarbeitung an oder in einer Prozessstation in der jeweiligen Öffnung oder Aufnahme in Längsrichtung in eine angehobene Position verschoben werden,
wobei die Behälter in der angehobenen Position weiterhin in den Öffnungen oder Aufnahmen des Trägers gehalten werden, um an oder in der Prozessstation behandelt oder verarbeitet zu werden, oder
wobei die Behälter in der angehobenen Position weiterhin in den Öffnungen oder Aufnahmen des Trägers aufgenommen sind, jedoch auf einer zusätzlichen Abstützfläche abgestützt werden oder mittels einer zusätzlichen Halte- oder Greifeinrichtung gehalten werden, um an oder in der Prozessstation behandelt oder verarbeitet zu werden.

6. Verfahren nach einem der vorhergehenden Ansprüche, wobei den Öffnungen oder Aufnahmen des Trägers Haltemittel zugeordnet sind, welche die Mehrzahl von Behältern (2) an dem Träger an den vorbestimmten Positionen und in der regelmäßigen zweidimensionalen Anordnung halten, wobei die Haltemittel die Behälter (2) an dem Träger (25; 134) formschlüssig halten.

7. Verfahren nach Anspruch 6, wobei der Träger (25) eine Mehrzahl von sich parallel erstreckenden und unter regelmäßigen Abständen zueinander angeordneten Querstegen (165) umfasst, entlang denen unter regelmäßigen Abständen zueinander eine Mehrzahl von Paaren von elastischen, konkav gewölbten Haltearmen (166) angeordnet sind, welche den oberen Rand (6) der von einem Paar von Haltearmen aufgenommenen Behälter (2) jeweils formschlüssig fixieren, wobei die Behälter unter Aufspreizung der Haltearme in diese eingeführt oder aus diesen abgezogen werden.

8. Verfahren nach Anspruch 6, wobei die Haltemittel zumindest zwei Haltezungen (140) umfassen, die am Rand einer jeweiligen Öffnung oder Aufnahme vorgesehen sind und von einer Oberseite des Trägers (134) abragen, um den jeweiligen Behälter zu halten, wobei die Haltezungen (140) beim Einführen der Behälter in die Öffnungen oder Aufnahmen elastisch weggeschwenkt oder weggeklappt werden, und wobei die Haltezungen (140) die Behälter mit radialem Spiel halten, wobei die Haltezungen (140) bevorzugt elastische Haltezungen sind.

9. Verfahren nach Anspruch 8, wobei die Haltezungen (140) die Behälter so halten, dass die Behälter (2) mit einem verbreiterten Rand (6), der an einem oberen Ende der Behälter ausgebildet ist, lose auf Oberseiten der Haltezungen (140) aufliegen, oder wobei die Haltezungen (140) einen verbreiterten Rand (6), der an einem oberen Ende der Behälter ausgebildet ist, so umgreifen, dass die Behälter mit radialem Spiel oder mit radialem und axialem Spiel von den Haltezungen gehalten werden.

10. Verfahren nach Anspruch 9, wobei am oberen Ende der Haltezungen (140) Einführschrägen (140d) ausgebildet sind, welche jeweils in eine von den Haltezungen radial einwärts vorstehende Haltenase (140e) zum Halten der Behälter übergehen.

11. Verfahren nach einem der Ansprüche 1 bis 5, wobei den Öffnungen oder Aufnahmen des Trägers Haltemittel zugeordnet sind, welche die Mehrzahl von Behältern (2) an dem Träger an den vorbestimmten Positionen und in der regelmäßigen zweidimensionalen Anordnung halten, wobei die Haltemittel die Behälter (2) an dem Träger (25; 134) reibschlüssig halten.

12. Verfahren nach Anspruch 11, wobei die Haltemittel als umlaufende und sich in Längsrichtung der Behälter erstreckende Aufnahmen (39; 65) ausgebildet sind und die Seitenwandabschnitte (4) der Behälter (2) zumindest abschnittsweise reibschlüssig umgreifen, wobei die Aufnahmen einen vieleckigen Querschnitt aufweisen, insbesondere einen viereckigen oder sechseckigen Querschnitt, wobei der Reibschluss durch das Zusammenwirken von einander gegenüberliegenden Seitenwänden und der zylindrischen Seitenwand (4) der Behälter bewerkstelligt wird.

13. Verfahren nach Anspruch 12, wobei sämtliche Seitenwände der Öffnungen oder Aufnahmen zwischen einer ersten Stellung und einer zweiten Stellung koordiniert verstellbar sind, wobei die Behälter in der ersten Stellung mit geringem Kraftaufwand in die Öffnungen oder Aufnahmen einführbar oder in diesen verschiebbar sind und die Behälter in der zweiten Stellung reibschlüssig in den Öffnungen oder Aufnahmen fixiert werden.

14. Verfahren nach Anspruch 13, wobei der Träger (25) in der zweiten Stellung rechteckig oder quadratisch ist und die koordinierte Verstellbewegung eine Scherbewegung von einer ersten Stellung, in welcher der Träger in Draufsicht ein Parallelogramm ausbildet, in die zweite Stellung ist.

15. Verfahren nach einem der vorhergehenden Ansprüche, wobei zumindest einige der Prozessstationen unter sterilen Umgebungsbedingungen angeordnet und betrieben werden.

16. Vorrichtung zur Behandlung oder Verarbeitung von Behältern (2), die zur Aufbewahrung von Substanzen für medizinische, pharmazeutische oder kosmetische Anwendungen dienen oder diese enthalten, wobei die Behälter an einem Ende offen ausgebildet sind,
mit einer Fördereinrichtung (221); und
einer Mehrzahl von Prozessstationen, in oder an denen die Behälter (2) jeweils behandelt oder verarbeitet werden; wobei
die Vorrichtung zur Behandlung oder Verarbeitung von Behältern ausgelegt ist, sodass eine Mehrzahl von Behältern (2) von der Fördereinrichtung (221) zur Behandlung oder Verarbeitung automatisiert an den Prozessstationen vorbeigeführt werden oder diese durchlaufen, während diese von einem Träger (25; 134) gemeinsam in einer regelmäßigen zweidimensionalen Anordnung gehalten werden, wobei der Träger eine Mehrzahl von Öffnungen oder Aufnahmen (32; 39; 87; 120) aufweist, welche die regelmäßige Anordnung vorgeben, und wobei die Böden (3) der Behälter an deren geschlossenen Enden von einer ersten Seite des Trägers her frei zugänglich sind,
**dadurch gekennzeichnet, dass** eine Prozessstation ein Gefriertrockenschrank (220) mit zumindest einer Kühlfläche (223) ist, in welchem ein Gefriertroeknungsprozess ausgeführt wird, wobei die Böden (3) der Behälter während des Gefriertrocknungsprozesses in dem Gefriertrockenschrank (220) in einem unmittelbaren Kontakt zu der jeweiligen Kühlfläche (223) stehen, während die Behälter von dem Träger gehalten werden oder in den Öffnungen oder Aufnahmen des Trägers aufgenommen sind.

## Claims

1. A process for the treatment or processing of containers (2), which serve for storing substances for medical, pharmaceutical or cosmetic applications or contain such substances, wherein the containers are open at one end and are conveyed automatically, by means of a conveyor (221), to processing stations or pass them for the treatment or processing, in which process
a plurality of containers (2) are conveyed by the conveyor (221) while the containers supported by a supporting structure (25; 134) in a regular two-dimensional array, wherein the supporting structure comprises a plurality of openings or receptacles (32; 39; 87; 120), which define the regular array, and wherein the bottoms (3) of the containers are freely accessible at their closed ends from a first side of said supporting structure,
**characterized in that** one processing station is a freeze-dryer (220) having at least one cooling plane (223), in which a freeze-drying process is carried out, wherein the bottoms (3) of the containers are in direct contact with the respective cooling plane (223) during the freeze-drying process in said freeze-dryer while the containers are supported by the supporting structure or are accomodated in the openings or receptacles of the supporting structure.

2. The process according to claim 1, wherein the supporting structures (25; 134) are accommodated in transport or packaging containers (1) and are removed from the respective transport or packaging container and supported on the respective cooling plane (223) for carrying out the freeze-drying process and wherein the the supporting structures (25; 134) are accommodated in transport or packaging containers (1) during the treatment or processing in or at at least one of the processing stations.

3. The process according to any of the preceding claims, wherein edge portions (68) of said supporting structure are removed or pivoted away for reducing the base area of said supporting structure when the containers are treated or processed in or at said processing station or for carrying out the freeze-drying process.

4. The process according to any of the preceding claims, wherein respective directly adjacent supporting structures are coupled or connected with each other such that the respective supporting structure cannot be displaced in a longitudinal direction and/or transverse direction temporarily.

5. The process according to any of the preceding claims, wherein the containers (2) are displaced in the respective opening or receptacle in a longitudinal direction thereof to a raised position for the treatment or processing at or in the processing station,
wherein the containers continue to be held in the raised position in the openings or receptacles of the supporting structure to be treated or processed at or in the processing station, or
wherein the containers continue to be accommodated in the openings or receptacles of the supporting structure in the raised position, but are supported on an additional supporting surface or by an additional holding or gripping device to be treated or processed at or in the processing station.

6. The process according to any of the preceding claims, wherein holding means are associated with the openings or receptacles of the supporting structure, which hold the plurality of containers (2) on the supporting structure at the predetermined positions and in the two-dimensional array, wherein said holding means support the containers (2) on the supporting structure (25; 134) in a form-fitting manner.

7. The process of claim 6, wherein the supporting structure (25) comprises a plurality transverse webs (165) extending in parallel with each other and spaced apart from each other at regular intervals, along which a plurality of pairs of resilient, concavely shaped holding arms (166) are disposed at regular intervals which fix the upper rims (6) of the containers (2) accommodated by a pair of opposite holding arms (166) in a form-fitting manner, wherein the containers are inserted into or retracted from the holding arms while spreading the holding arms.

8. The process of claim 6, wherein the holding means comprises at least two resilient holding tongues (140), which are disposed at a rim of the respective opening or receptacle and protrude from an upper side of the supporting structure (134) for supporting the respective container, wherein the holding tongues (140) are resiliently pivoted or clapped away when the containers are inserted into the openings or receptacles and wherein the holding tongues (140) support the containers with a radial play, wherein the holding tongues (140) are preferably resilient holding tongues.

9. The process of claim 8, wherein the holding tongues (140) support the containers such that the containers (2) rest loosely on upper sides of the holding tongues (140) with an upper rim (6) formed at an upper end of the containers, or wherein the holding tongues (140) embrace an expanded rim (6) formed at an upper end of the containers such that the containers are supported by the holding tongues with a radial play or with a radial and axial play.

10. The process of claim 9, wherein slanted insertion surfaces (140d) are formed at upper ends of the holding tongues (140) each of which pass into a holding nose (140e) protruding radially inwards from the holding tongues for supporting the containers.

11. The process according to any of claims 1 to 5, wherein holding means are associated with the openings or receptacles of the supporting structure, which hold the plurality of containers (2) on the supporting structure at the predetermined positions and in the two-dimensional array, wherein said holding means are configured for holding the containers (2) on the supporting structure (25; 134) by friction.

12. The process of claim 11, wherein the holding means are formed as circumferential receptacles (39; 65) extending in the longitudinal direction of the containers and wherein side wall portions (4) of the containers (2) are embraced at least partially to accomplish said friction, wherein the receptacles have a polygonal cross-section, in particular a quadrangular or hexangular cross-section, wherein the frictional engagement is accomplished by the interaction of opposing side walls of said receptacles and the cylindrical side walls (4) of the containers

13. The process of claim 12, wherein all side walls of the openings or receptacles can be adjusted in a coordinated manner all-together between a first position and a second position, wherein, in the first position, the containers can be inserted with little force into the openings or receptacles or can be displaced therein, and wherein, in the second position, the containers are fixed by friction in the openings or receptacles.

14. The process of claim 13, wherein the supporting structure (25) is rectangular or square-shaped in the second position and wherein the coordinated adjustment movement is a shearing movement from a first position, in which the supporting structure forms a parallelogram in a plan view, to the second position

15. The process of any of the preceding claims, wherein at least some of the processing stations are disposed and operated under sterile environmental conditions..

16. An apparatus for the treatment or processing of containers (2), which serve for storing substances for medical, pharmaceutical or cosmetic applications or contain such substances, wherein the containers are open at one end, comprising
a conveying device (221); and
a plurality of processing stations, in or at which the respective containers (2) are treated or processed; wherein
the apparatus is configured for the treatment or processing of the containers such that a plurality of containers (2) are conveyed automatically, by means of said conveying device (221), to said processing stations or pass them to be treated or processed while these are supported all together by a supporting structure (25; 134) in a regular two-dimensional array, wherein the supporting structure has a plurality of openings or receptacles (32; 39; 87; 120), which together determine the regular array, and wherein the bottoms (3) of the containers are freely accessible at their closed ends from a first side of said supporting structure, and
**characterized in that** one processing station is a freeze-dryer (220) having at least one cooling plane (223), where a freeze-drying process is carried out, wherein the bottoms (3) of the containers are in direct contact with the respective cooling plane (223) during the freeze-drying process in said freeze-dryer (220) while the containers are supported by the supporting structure or are accommodated in the openings or receptacles of the supporting structure.

## Revendications

1. Procédé de traitement ou de transformation de récipients (2), utilisés pour le stockage de substances pour des applications cosmétiques, médicales, pharmaceutiques ou pour contenir de telles substances, dans lequel les récipients sont conçus pour être ouverts à une extrémité et convoyés automatiquement, au moyen d'un convoyeur (221), vers des stations de traitement ou devant être passés pour traitement ou transformation, dans lequel procédé
une pluralité de récipients (2) sont convoyés par le convoyeur (221) alors que les récipients sont soutenus par une structure de support (25; 134) dans une matrice bidimensionnelle régulière, dans lequel la structure de support a une pluralité d'ouvertures ou de sièges (32; 39; 87; 120) définissant une matrice régulière, et dans lequel les fonds (3) des récipients sont librement accessibles à leurs extrémités fermées depuis un premier côté de ladite structure de support,
**caractérisé en ce que** une station de traitement est un séchoir à lyophilisation (220) ayant une surface de refroidissement (223), dans lequel un procédé de lyophilisation est effectué, dans lesquels les fonds (3) des récipients sont en contact direct avec la surface de refroidissement respective (223) durant le procédé de lyophilisation au sein du séchoir par lyophilisation tandis que les récipients sont soutenus par la structure de support ou sont disposées dans les ouvertures ou les sièges de la structure de support.

2. Le procédé selon la revendication 1, dans lequel les structures de support (25; 134) sont disposés dans des récipients de transport ou de conditionnement (1) et sont enlevés du récipient de transport ou de conditionnement respectif et soutenus sur la surface de refroidissement respective (223) pour mettre en oeuvre le procédé de lyophilisation et dans lequel les structures de support (25 ; 134) sont disposées dans des récipients de transport ou de conditionnement (1) durant le traitement ou la transformation ou au moins dans une des stations de traitement.

3. Le procédé selon l'une quelconque des revendications précédentes, dans lequel les parties de bord (68) de ladite structure de support sont retirées ou écartées par pivotement afin de réduire la surface du support de ladite structure de support lorsque les récipients sont traités ou transformés dans ou sur le poste de traitement ou pour mettre en oeuvre le procédé de lyophilisation.

4. Le procédé selon l'une quelconque des revendications précédentes, dans lequel chacun des supports adjacents sont directement couplés ou reliés les uns aux autres directement, de telle manière à ce que les supports respectifs ne peuvent être provisoirement déplacés suivant une direction longitudinale et/ou une direction transversale.

5. Le procédé selon l'une quelconque des revendications précédentes, dans lequel les récipients (2) sont déplacés dans les ouvertures ou sièges respective suivant une direction longitudinale vers une position relevée pour un traitement ou une transformation au sein de la station de traitement,
dans lequel les récipients continuent d'être maintenus en position relevé dans les ouvertures ou les sièges de la structure de support pour y être traités ou transformée ou dans la station de traitement, ou
les récipients continuent à être reçus dans les ouvertures ou les sièges de la structure de support dans une position relevée, mais sont soutenus par une surface de soutien additionnelle ou par un dispositif de maintien ou de préhension additionnel pour le traitement ou la transformation ou dans la station de traitement.

6. Le procédé selon l'une quelconque des revendications précédentes, dans lequel les moyens de maintien sont associés aux ouvertures ou aux sièges de la structure de support, lesquels maintiennent la pluralité de récipients (2) sur la structure de support à des positions prédéterminées et au sein de la matrice à deux dimensions, dans lequel lesdits moyens de maintien soutiennent les récipients (2) sur la structure de support (25 ; 134) avec une forme appropriée.

7. Le procédé de la revendication 6, dans lequel la structure de support (25) comporte une pluralité d'entretoises transversales (165) s'étendant parallèlement les unes aux autres et disposées à des distances régulières les unes des autres, le long desquelles une pluralité de paires de bras de support (166) élastiques, de forme concave, sont disposées à des intervalles réguliers pour fixer les bords supérieurs (6) des récipients (2) reçus par une paire de bras de support opposés (166) suivant une forme appropriée, dans lequel les récipients sont insérés ou retirés par les bras de support pendant l'écartement des bras de support.

8. Le procédé selon la revendication 6, dans lequel les moyens de maintien comprennent au moins deux languettes de retenue (140) qui sont prévus au bord d'une ouverture ou d'un siège respectif et faisant saillie depuis un bord supérieur de la structure de support (134) pour supporter le récipient respectif, dans lequel les languettes de retenue (140) sont pivotées de manière élastique ou écartées élastiquement lorsque les récipients sont insérés dans les ouvertures ou les sièges et dans lequel les languettes de retenue (140) soutiennent les récipient avec un jeu radial, dans lequel les languettes de retenue sont de préférence des languettes de retenue élastiques.

9. Le procédé selon la revendication 8, dans lequel les languettes de retenue (140) soutiennent les récipient de telle manière à ce que les récipients (2) reposent lâchement sur les bords supérieurs des languettes de retenue (140) avec un bord supérieur (6) formé à une extrémité supérieure des récipients, ou dans lequel les languettes de retenue (140) entourent un bord étendu (6) formé à une extrémité supérieure des récipient de telle façon que les récipients sont soutenus par les languettes de retenue avec un jeu radial ou avec un jeu radial et axial.

10. Le procédé de la revendication 9, dans lequel des surfaces d'insertion en biseau (140d) sont formées aux extrémités supérieures des languettes de retenue (140), chacune passant dans un patte de retenue (140^{e}) faisant saillie radialement vers l'intérieur des languettes de retenue pour le soutien des récipients.

11. Le procédé selon l'une quelconque des revendications 1 à 5, dans lequel les moyens de maintien sont associés à des ouvertures ou sièges de la structure de support, qui maintient la pluralité de récipients (2) sur la structure de support à des positions prédéterminées et au sein de la matrice bi-dimensionnelle, dans lequel lesdits moyens de maintien sont configurés pour maintenir les récipients (2) sur la structure de support (25 ; 134) par friction.

12. Le procédé de la revendication 11, dans lequel les moyens de maintien prennent la forme de sièges circonférentiels (39, 65) s'étendant suivant une direction longitudinale des récipients et dans lequel les parties de parois latérales (4) des récipients (2) sont entourées au moins partiellement pour ladite friction, dans lequel les sièges présentent une section transversale polygonale, en particulier une section transversale quadrangulaire ou hexagonale, dans lequel la friction est réalisée par l'interaction de parois latérales opposées desdits récipients et des parois latérales cylindriques (4) des récipients.

13. Le procédé de la revendication 12, dans lequel toutes les parois latérales des ouvertures ou des sièges peuvent être ajustées ensemble de manière coordonnées entre une première position et une seconde position, dans lequel, dans la première position, les récipients peuvent être insérés a force réduite dans les ouvertures ou les sièges ou peuvent y être déplacées, et dans lequel, dans la seconde position, les récipients sont fixés par friction dans les ouvertures ou les sièges.

14. Le procédé de la revendication 13, dans lequel la structure de support (25) est rectangulaire ou de forme carrée dans la seconde position et dans lequel le mouvement d'ajustement coordonné est un mouvement de cisaillement depuis la première position, dans lequel la structure de support formes un parallélogramme dans une vue plane, vers la seconde position.

15. Le procédé selon l'une quelconque des revendications précédentes, dans lequel au moins une partie des stations de traitement sont disposées et exploitées dans des conditions environnementales stériles.

16. Un appareil pour le traitement ou la transformation de récipients (2), utilisés pour le stockage de substances pour des applications cosmétiques, médicales ou pharmaceutiques ou pour contenir de telles substances, dans lequel les récipients sont ouverts à une extrémité, comprenant :
un dispositif de convoyage (221) ; et
une pluralité de stations de traitement, dans lesquelles ou au travers desquelles les récipients (2) sont traités, dans lequel
l'appareils est configuré pour le traitement ou la transformation des récipients de telle façon qu'une pluralité de récipients (2) est convoyée automatiquement, au moyen du dispositif de convoyage (221), vers les dites stations de traitement ou pour les traverser en vue d'un traitement alors qu'ils sont soutenus ensemble par une structure de support (25 ; 134) dans une matrice bi-dimensionnelle régulière, dans lequel la structure de support présente une pluralité d'ouvertures ou de sièges (32; 39 ; 87 ; 120) qui déterminent ensemble la matrice régulière, et dans lequel les fonds (3) des récipients sont librement accessibles à leurs extrémités fermées depuis un premier côté de ladite structure de support, et
**caractérisé en ce que** une station de traitement est un séchoir à lyophilisation (220) ayant une surface de refroidissement (223), dans lequel un procédé de lyophilisation est effectué, dans lesquels les fonds (3) des récipients sont en contact direct avec la surface de refroidissement respective (223) durant le procédé de lyophilisation au sein du séchoir par lyophilisation tandis que les récipients sont soutenus par la structure de support ou sont disposées dans les ouvertures ou les sièges de la structure de support.
